# EUROPEAN PATENT APPLICATION

(11) **EP 4 535 367 A1**
(43) Date of publication of application: **09.04.2025**
(21) Application number: 24200784.7
(22) Date of filing: 17.09.2024
(51) Int. Cl.: G16H 15/00, G16H 10/00, G06F 40/30, G06F 40/166, G06F 3/023

(54) **SYSTEMS AND METHODS FOR INTELLIGENT MEDICAL EDITORS**

(30) Priority: 06.10.2023 IN 202341066971
(71) Applicant: GE Precision Healthcare LLC, Waukesha, WI 53188 (US)
(72) Inventor: SASIDHARAN, Sanand, 560066 Bengaluru (IN); ACHARA, Akshit, 560066 Bengaluru (IN); KANAMARLAPUDI, Anuradha, 560066 Bengaluru (IN); GORAVAR, Shivappa, 560066 Bengaluru (IN); BHOI, Kshireswar, 560066 Bengaluru (IN)
(74) Representative: Fennell, Gareth Charles

(57) **Abstract**

Various systems and methods are provided for intelligent medical editors. An intelligent medical editor may edit medical materials such as electronic medical records, medical reports, and treatment plans. An intelligent medical editor may provide edit recommendations and suggested additions to medical materials to a user of the editor. Edit recommendations and suggested additions may be related to contradictions, incompleteness, clarity, and clinical guidelines.

## Description

### TECHNICAL FIELD

The subject matter disclosed herein relates generally to healthcare technology, specifically for technology assisted editing of medical reports and records.

### BACKGROUND

Medical reports, documents, and records are an important part of healthcare around the world. These may be radiology reports, lists of chronic health conditions, medication lists, imaging results, diagnostic reports, department reports, clinical notes, staff action orders, and the like. Information in the medical reports, documents, and records are used to make important decisions affecting human health.

Currently, many negative issues affect the editing and updating of such medical materials. Ambiguity of meaning and word usage may exist instead of clarity. Ambiguity may include poor phasing, or different types of phrasing for the same issue across medical professions in different regions or languages. Contradictions inside a record or between different records for a given patient may exist instead of correctness. Incomplete materials or information may cause issues, instead of full and complete data. Information may be missed, instead of captured. For example, a lesion in a medical image is not found by a radiologist reviewing a Computed Tomography (CT) scan. Further, inconsistencies of information between the medical materials and medical standards or guidelines may lead to legal or medical issues. And, of course, information may just be wrong instead of right. Human-made errors may happen when writing medical reports and editing medical records. Spelling mistakes can change the meaning of information and affect patient care - for example affecting a dosage of medication for a patient. Accurate information that is helpful for clinical decision making is important. Systems and methods are needed to improve such issues within medical materials.

Not only can the medical materials have issues themselves, but it can take a long time and substantial human effort to generate, edit, and correct the materials. Clinicians, doctors, physicians, nurses, and other healthcare professionals are highly paid and only have a limited amount of time. They usually can best use that time serving patients instead of updating medical reports, documents, and records. Systems and methods are needed to speed up the time users take to edit and update medical materials and save money for the stakeholders involved.

Medical professionals these days also have to deal with many computer and information technology (IT) systems along the patient journey. A medical professional may be required to use multiple tools on different platforms during the course of a patient journey and manually work with IT systems, which include Electronic Medical Records (EMR) and Electronic Health Records (EHR). These varying IT systems may have gaps in compatibility and increase the burden on a medical professional, especially if the professional needs to provide a comprehensive multi-modal patient analysis. Systems and methods are needed to unify medical material editing systems both in compatibility and usability.

Ensuring the correctness, completeness and clarity of EMRs is paramount to improvement of clinical outcomes. Imprecise and incomplete EMRs, and other medical materials, adversely affect the speed and quality of all the subsequent clinical decisions such as diagnosis/treatment decisions, clinical trial selection, referencing guidelines and case studies.

Medical materials, such as the reports, records, and documents mentioned above, also may have the issue of personalization bias. Instead of reports being standard across a hospital or industry, reports can be too specific to a department or individual. Systems and methods are needed to help generate industry standard medical materials.

### SUMMARY

This summary introduces concepts that are described in more detail in the detailed description. It should not be used to identify essential features of the claimed subject matter, nor to limit the scope of the claimed subject matter.

In an aspect, medical systems and methods are proposed that may include one or more processors; memory; and one or more programs, wherein the one or more programs are stored in the memory and configured to be executed by the one or more processors, the one or more programs including instructions for: identifying a plurality of textual entities in a free-text medical report in real time as a user is writing the report; suggesting edit recommendations related to the textual entries to the user, where the edit recommendations are generated by one or more artificial intelligence modules; and wherein the edit recommendation related to probable errors or improvements based on data comprising patient history, clinical care guidelines, and medical ontologies. The medical systems and methods may also include retraining the artificial intelligence module using the medical report and the user's selections related to the edit recommendations as a training data.

The errors or improvements may comprise clinical incorrectness where the report contains findings on absent or incorrect anatomy of the patient, a treatment plan that is incompatible with the clinical care guidelines, information in the report about prior medical appointments or prior medical exams that is incomplete, detected differences between the report and a related synoptic reporting standard related to the report type, suggested links to source data from the patient EMR or patient images stored in a PACS.

Edit recommendations may be presented on a user interface via a text overlay near the textual entry in the report. Edit recommendations may be presented on a user interface via a chatbot supported by generative artificial intelligence and large language models.

In an aspect, medical systems and methods are proposed that may include a device having one or more processors and memory, including steps of: identifying a plurality of textual entries in a free text medical report; generating suggestions with smart modules; providing suggested content on a user interface related to the patient and medical report type; providing suggestions to the user related to the textual entries and other report content as the user is editing the report; providing warnings about probable errors based on at least one of patient history, clinical care guidelines, or medical ontologies; and updating the medical report based on the user chosen selections to the suggested content. Smart modules can be at least one of a clinical information extraction module, an enriched information overlay module, a contradiction and incompleteness detection module, an edit recommendation module, a language module finetuning module, and a natural language understanding updation module.

### BRIEF DESCRIPTION OF DRAWINGS

Numerous aspects, implementations, objects and advantages of the present invention will be apparent upon consideration of the following detailed description, taken in conjunction with the accompanying drawings, in which like reference characters refer to like parts throughout, and in which:
FIG. 1 shows stages in a patient journey, according to an embodiment.
FIG. 2A shows a hardware device for a healthcare environment, according to an embodiment.
FIG. 2B shows a block diagram for a computed tomography hardware and software system, according to an embodiment.
FIG. 3 shows a block diagram for an intelligent medical editor, according to an embodiment.
FIG. 4 shows a flow diagram for an intelligent medical editor, according to an embodiment.
FIG. 5 shows a natural language processing (NLP) model update module, according to an embodiment.
FIG. 6 shows a natural language generation (NLG) module with a natural word prediction (NWP) module, according to an embodiment.
FIG. 7 shows a block diagram supporting natural report generation, according to an embodiment.
FIG. 8A shows a local model for language fine tuning, according to an embodiment.
FIG. 8B shows details for a language fine tuning module, according to an embodiment.
FIG. 9 shows a clinical information extraction module, according to an embodiment.
FIG. 10 shows an edit recommendation module, according to an embodiment.
FIG. 11 shows a contradiction and incompleteness detection module, according to an embodiment.
FIG. 12 shows a clinical care guidelines deviation flow, according to an embodiment.
FIG. 13 shows a user interface with suggested edits for a digital document, according to an embodiment.
FIG. 14 shows details for an enriched information overlay module, according to an embodiment.
FIG. 15 shows a comparison between a standard report and a suggestions-supported report, according to an embodiment.
FIG. 16 shows a user interface with suggested edits to a digital document, according to an embodiment.
FIG. 17 shows a user interface with medical corrections in a digital document, according to an embodiment.
FIG. 18 shows a block diagram related to edit recommendations leveraging predictive and generative models, according to an embodiment.
FIG. 19 shows a flow chart and block diagram related to patient profiling, according to an embodiment.
FIG. 20 shows a flow chart for updating a medical report based on selected suggested edits, according to an embodiment.
FIG. 21 shows a schematic block diagram of a sample-computing environment, according to an embodiment.
FIG. 22 shows a schematic block diagram of another sample-computing environment, according to an embodiment.
FIG. 23 shows a schematic block diagram of another sample-computing environment, according to an embodiment.
FIG. 24 shows a schematic block diagram of another sample-computing environment, according to an embodiment.
FIG. 25 shows a schematic block diagram of another sample-computing environment, according to an embodiment.
FIG. 26 shows a schematic block diagram illustrating a suitable operating environment, according to an embodiment.

### DETAILED DESCRIPTION

In the following detailed description, reference is made to the accompanying drawings that form a part hereof, and in which is shown by way of illustration specific examples that may be practiced. These examples are described in sufficient detail to enable one skilled in the art to practice the subject matter, and it is to be understood that other examples may be utilized, and that logical, mechanical, electrical and other changes may be made without departing from the scope of the subject matter of this disclosure. The following detailed description is, therefore, provided to describe an exemplary implementation and not to be taken as limiting on the scope of the subject matter described in this disclosure. Certain features from different aspects of the following description may be combined to form yet new aspects of the subject matter discussed below.

When introducing elements of various embodiments of the present disclosure, the articles "a," "an," "the," and "said" are intended to mean that there are one or more of the elements. The terms "comprising," "including," and "having" are intended to be inclusive and mean that there may be additional elements other than the listed elements.

In an embodiment, systems and methods are proposed for EMR editing assistance throughout the patient journey and numerous downstream analyses leveraging, but not limited to, standardized EMRs, clinician interactions and multi-modal patient history. This solves multiple problems caused by varying EMR reporting standards by providing correctness, completeness and clarity of EMRs. While clinician benefits from the AI assistance, the feedback from the interactions is used to improve the AI assistance itself. The improvements are done in a federated way without compromising the patient privacy.

A number of technical effects are produced by the systems and methods herein, including identifying edit-candidates using masked and causal Large Language Models (LLM), factual contradiction and incompleteness detection using patient data model, clinical guidelines and medical ontologies, incompleteness detection and report structure recommendation using synoptic reporting standards, cross-reference recommendation, providing a standard tool for writing medical reports providing the suggesting the alternative words using medical standards vocabulary, and linking using patient data model and clinical guidelines, and re-aligning reports to different templates using Large Language Models (LLM). Further technical effects produced by the systems and methods herein include automatically identifying missing body parts (due to biological sex or a past surgery where the body part was removed), e.g., a male patient is diagnosed with ovarian cancer, and medical guideline compatibility detection. For example, warnings can be popped up if a treatment decision is taken which is not present in any of the treatment options suggested by care guidelines.

Medical text processing is an important application of natural language processing (NLP) and supervised machine learning is used to process the medical text to extract useful information. Supervised machine learning techniques require annotated text to learn from the data. All the useful words need to be labelled with the most appropriate label out of all the possible class labels. The annotated text needs to be in a format which can represent the word-label relations and other representations based on the NLP task. Some of the common formats include XMI (XML metadata exchange) and CoNLL. The CoNLL format is a text file with one word per line with sentences separated by an empty line.

The time taken and redundancy can be reduced with the help of a smart annotation and editing tool which can help annotate the reports while writing the report itself by providing label suggestions for each word. This requires an artificial intelligence (AI) enabled report writing tool, which needs to be trained with limited data. The tool helps us to get annotated data quickly. The model can be tuned at the end of each report by comparing the predictions with actual annotation selection by doctors there by improving the adaptability of model for specific doctor or type of report.

In an embodiment, an intelligent medical editor can be very impactful as multiple modules can be integrated into the tool that can provide intelligence based on the domain. This will help medical health professionals get annotated data quickly to perform NLP tasks in different domains. In an embodiment, an intelligent medical editor also helps to bring a standard form of reporting and brining similarity in the wide variety of reports. This is beneficial to training the model with lesser data.

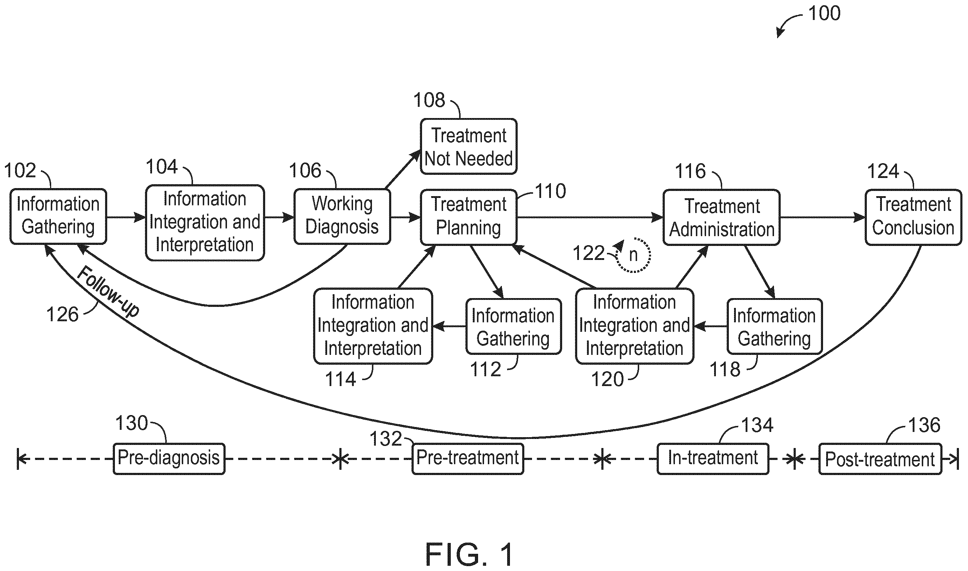

FIG. 1 shows stages in a patient journey, according to an embodiment. FIG. 1 includes patient journey 100 with steps of information gathering 102, information integration and interpretation 104, working diagnosis 106, treatment not needed 108, treatment planning 110, information gathering 112, information integration and interpretation 114, treatment administration 116, information gathering 118, information integration and interpretation 120, repeat process 122, treatment conclusion 124, and follow-up 126. Patient journey 100 also has patient stages of pre-diagnosis 130, pre-treatment 132, in-treatment 143, and post-treatment 146.

An initial stage of a patient journey is pre-diagnosis 130. This includes the initial information gathering 102 before or during an initial medical appointment. This information may be data, observations, measurements, images, test results, medical opinions, evidence, or other information the patient or initial consult medical provider gathers. In this stage, patients seek care for their symptoms with a provider, often a primary care physician or other medical generalist. And later in patient journey stages, specialists may be consulted. Sometimes a patient directly seeks out a specialist to evaluate their condition, which may or may not shorten their journey depending on their choice of an appropriate specialist. Information gathering 102 may be from a variety of information gathering modes, such as clinical history and interview, physical examinations, diagnostic testing, consultation with experts, and the like.

Steps in the patient journey are enabled by medical reports and records such as EMRs, Radiology Information Systems (RIS), radiology reports, and other medical types of reports and records. Information may be integrated in step 104 into an EMR, which may include gathered evidence, reasonings and thought processes, conclusions, prescriptions for further tests, prescriptions for treatment, treatment planning thought process, treatment progress notes, treatment response notes, and follow up notes.

Information integration and interpretation steps 104, 114, and 120 may be enabled by an intelligent medical editor, as described herein throughout. Information can be analyzed by an intelligent medical editor and then presented with suggestions for integration and interpretation to speed up and improve information integration and interpretation steps 104, 114, and 120. Such intelligent medical editors can provide auto-creation of reports as well as automatic suggestions and edit recommendations.

During an initial consultation with a medical provider, an initial working diagnosis 106 is developed, and may be put into an intelligent medical editor. Sometimes treatment may not be needed as a medical issue may be resolved on its own, and the patient journey ends at treatment not needed 108. Other times, more information is gathered at information gathering 102. And many times, an initial treatment is planned at treatment planning 110. Treatment planning 110 may be done with the initial medical provider, along with nurses, specialists, and the patient. An intelligent medical editor can suggest treatments based on medical guidelines.

During treatment planning 110, a medical provider works with a number of different types of information and medical devices to continue to perform information gathering 112. And the information gathered feeds into information integration and interpretation 114. In an embodiment, this is done with a computing device running an intelligent medical editor. These steps may be done in pre-treatment phase 132 of a patient journey.

In-treatment phase 134 of the patient journey may include treatment administration 116. Treatment administration 116 may include many types of treatment based on the symptoms and underlying medical issue, such as watch and wait, chemotherapy or other drug therapies, radiation therapy, immunotherapy, vaccine therapy, stem cell transplantation, blood transfusion, palliative care, physical therapy, and other medical treatments. A health system may provide a patient with both on-site and follow-up care (prescriptions, physical therapy, counseling, or suggested lifestyle changes).

In-treatment phase 134 of the patient journey also includes further information gathering 118, information integration and interpretation 120, and potentially repeating the process 122. Repeating the process may be done as needed, because this phase of the diagnostic journey may include involves one or multiple evaluations by specialists. For example, a patient presenting with fever of unknown origin may be referred by their primary care physician to a rheumatologist and infectious disease specialist simultaneously or in sequence. This phase may be expedited in inpatient settings where multi-specialty consultation is available, particularly at academic medical centers.

Post-treatment phase 136 of the patient journey may include treatment conclusion 124 and follow-up 126. Treatment conclusion 124 occurs when a medical condition is at the point of taking a break or being complete with treatment. The patient manages his or her care between clinical visits; meanwhile, the health system fosters engagement between the patient and physician in order to enable the patient to address symptoms and maintain good health. This patient follow up and retention phase is a crucial part of the patient journey that is often forgotten after a patient receives treatment. Getting feedback through follow up 126 is necessary so the system can gather insights into patient experiences and satisfaction. The patient's input can then be used to improve patient journey mapping, which ultimately helps to improve the patient experience, quality of care and process efficiency.

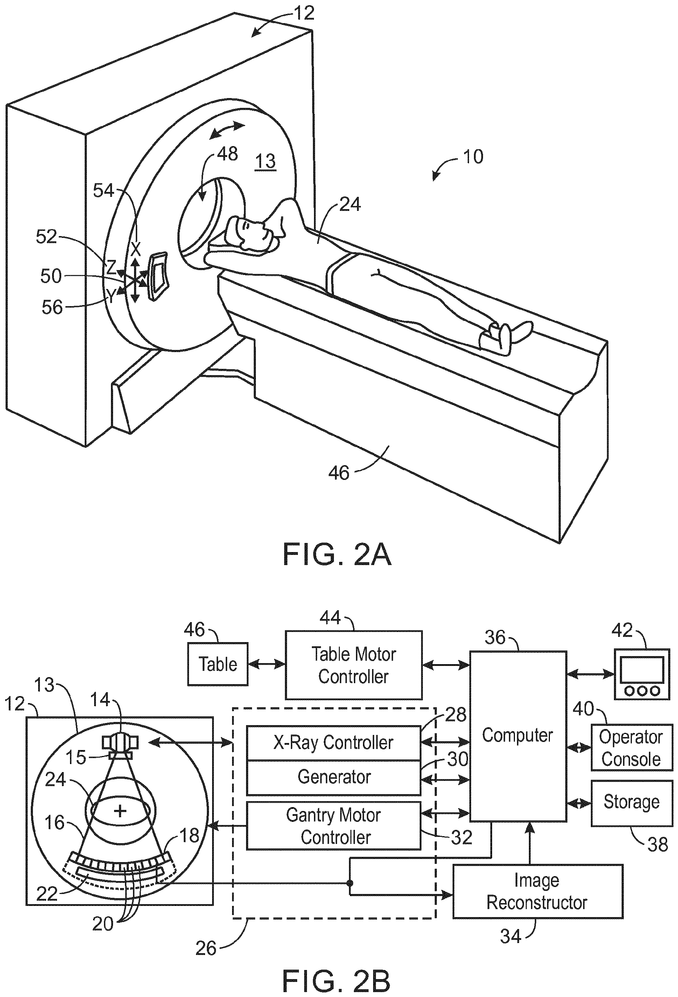

FIG. 2A shows a hardware device for a healthcare environment, according to an embodiment. FIG. 2B shows a block diagram for a computed tomography hardware and software system, according to an embodiment. Such machines may be used as medical imaging devices along a patient journey.

FIGs. 2A and 2B show a computed tomography (CT) imaging system 10 including a gantry 12. Gantry 12 has a rotary member 13. An x-ray source 14 that projects a beam of x-rays 16 through pre-patient collimator 15 toward a detector assembly 18 on the opposite side of the rotary member 13. X-ray source 14 may also be referred to as x-ray tube or x-ray generation component. X-ray source 14 is a type of emissions component. A main bearing may be utilized to attach the rotary member 13 to the stationary structure of the gantry 12. Detector assembly 18 is formed by a plurality of detectors 20 and data acquisition systems (DAS) 22, and can include a post-patient collimator. The plurality of detectors 20 sense the projected x-rays that pass through a subject 24, and DAS 22 converts the data to digital signals for subsequent processing. Each detector 20 produces an analog or digital electrical signal that represents the intensity of an impinging x-ray beam and hence the attenuated beam as it passes through subject 24. During a scan to acquire x-ray projection data, rotary member 13 and the components mounted thereon can rotate about a center of rotation.

Rotation of rotary member 13 and the operation of x-ray source 14 are governed by a control mechanism 26 of CT system 10. Control mechanism 26 can include an x-ray controller 28 and generator 30 that provides power and timing signals to x-ray source 14 and a gantry motor controller 32 that controls the rotational speed and position of rotary member 13. An image reconstructor 34 receives sampled and digitized x-ray data from DAS 22 and performs high speed image reconstruction. The reconstructed image is output to a computer 36 which stores the image in a computer storage device 38.

Computer 36 also receives commands and scanning parameters from an operator via operator console 40 that has some form of operator interface, such as a keyboard, mouse, touch sensitive controller, voice activated controller, or any other suitable input apparatus. Display 42 allows the operator to observe the reconstructed image and other data from computer 36. The operator supplied commands and parameters are used by computer 36 to provide control signals and information to DAS 22, x-ray controller 28, and gantry motor controller 32. In addition, computer 36 operates a table motor controller 44 which controls a motorized table 46 to position subject 24 and gantry 12. Particularly, table 46 moves a subject 24 through a gantry opening 48, or bore, in whole or in part. A coordinate system 50 defines a patient or Z-axis 52 along which subject 24 is moved in and out of opening 48, a gantry circumferential or X-axis 54 along which detector assembly 18 passes, and a Y-axis 56 that passes along a direction from a focal spot of x-ray tube 14 to detector assembly 18.

Such imaging systems provide data, images, and information for information gathering stage and interpretation along the patient journey. Computer 36 may have an intelligent medical editor, in an embodiment, that provides a CT image along with a radiology report that was edited using systems such as disclosed within. Then the radiology report may be further integrated into a larger EMR using a different instance, or the same, intelligent medical editor.

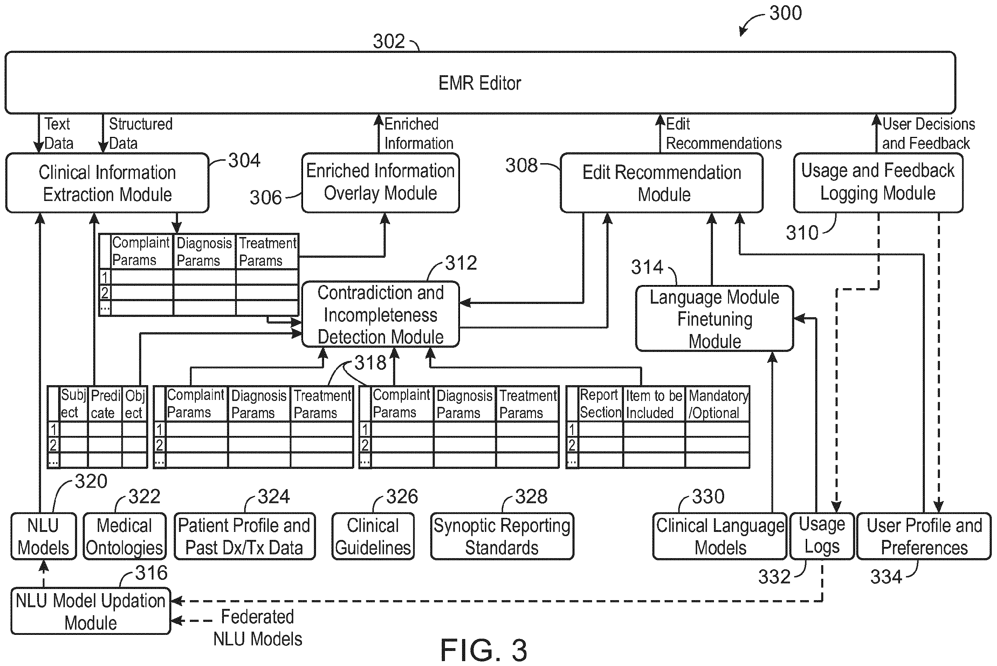

FIG. 3 shows a block diagram for an intelligent medical editor, according to an embodiment. FIG. 3 includes medical system 300, EMR editor 302, clinical information extraction module 304, enriched information overlay module 306, edit recommendation module 308, usage and feedback logging module 310, contradiction and incompleteness module 312, language model finetuning module 314, NLU model updation module 316, medical data structures 318, NLU models 320, medical ontologies 322, patient profile and past Dx/Tx data 324, clinical guidelines 326, synoptic reporting standards 328, clinical language models 330, usage logs 332, and user profile and preferences 334. Medical system 300 may be an intelligent medical editor, or parts of medical system 300 may be the intelligent medical editor, in varying embodiments. Individual modules may be within the intelligent medical editor software or in other connected software programs. They may be separate modules within a larger program, or separate programs linked together, such as by procedure calls. FIG. 3 indicates the functional components of a medical system 300 including an intelligent medical editor.

EMR editor 302 is an example editor for electronic medical records. In other embodiments, the editor may be editing reports, records, and other medical materials. EMR editor 302 provides a user interface that interacts with users, both presenting information to, and receiving information from, users. EMR editor 302 is connected to a data storage system where the EMR records are stored for updating. EMR editor 302 may send text data and structured data to clinical information extraction module 304. EMR editor 302 may receive enriched information from enriched information overlay module 306. EMR editor 302 may receive edit recommendations from edit recommendation module 308. EMR editor 302 may receive user decisions and feedback from usage and feedback logging module. EMR editor may also send information to these modules based on user interactions. For example, if edit recommendation module 308 provides an edit suggestion and a user selects it, the response to that recommendation may go back to the edit recommendation module 308 for implementing the change as well as being input into usage logs 332 through usage and feedback logging module 310 to better adaptively learn user behavior - both individually to the specific user and generally to all users who had a similar suggestion presented on EMR editor 302.

EMR editor 302 can provide an initial EMR record or template report that is prepopulated with data, and then overlaid with suggestions and edit recommendations. And while a user is interacting with the EMR record or report, the system can continually adapt its suggestions and information in real time. This provides real-time assistance to medical professionals as they are working on creating and updating medical materials. In addition to suggestions and edit recommendations, the EMR editor can provide annotations of information, text, images, and other data objects while preparing the report. This helps to reduce the time to annotate the reports after completing the reports by doctors.

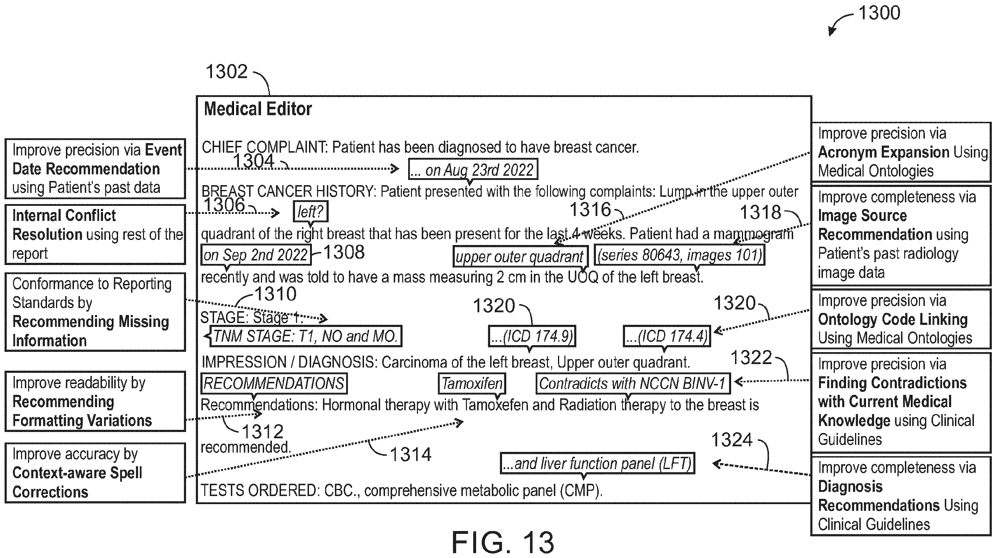

User interface examples are shown in, at least, FIGs. 13-17. These show examples of medical edit suggestions and recommendations an intelligent medical editor can provide, in various embodiments. All give a user a concise list of improvement edits. In some embodiments, a user can interact (such as a mouse click, finger touch, right click, finger swipe, or other inputs depending on the computer hardware and software involved) with the suggestions to learn more about why the suggestion was provided. In these circumstances the software can provide the medical reasoning, ontology, grammar, or other reasoning of why the suggestion was made so a user can have further confidence and understanding of the suggestion.

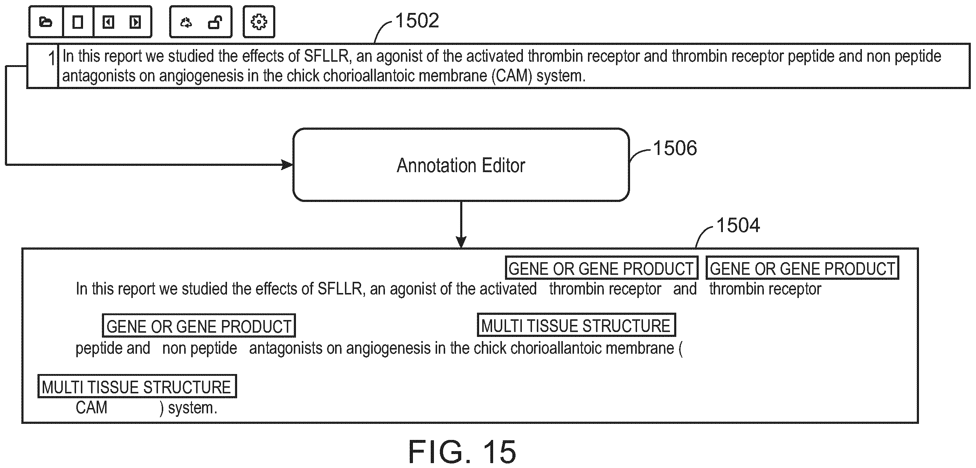

FIG. 15 shows a comparison between a standard report and a suggestions-supported report, according to an embodiment. FIG. 15 includes an example of an initial medical report 1502 that is input to an intelligent medical editor, such as annotation editor 1506. Annotation editor 1506 provides a user interface screen 1504 that includes the initial medical report 1502 and also provides five edit suggestions for a user to consider. A user can interact with the suggested edits in user interface screen 1504 to change, and potentially improve, initial medical report 1502.

Returning to FIG. 3, clinical information extraction module 304 may receive text data and structured data from EMR editor 302. Clinical information extraction module 304 may receive medical data structures 318 and NLU modules 320. Clinical information extraction module 304 may put information to storage data, including in medical data structures 318.

Clinical information extraction module 304 takes in data and information from a myriad of sources, some healthcare related some may not be. This is an important part of the system as it helps an intelligent medical editor be context aware. Information may be provided to and from NLU model updation module 316, medical data structures 318, NLU models 320, medical ontologies 322, patient profile and past Dx/Tx data 324, clinical guidelines 326, synoptic reporting standards 328, clinical language models 330, usage logs 332, and user profile and preferences 334. A context aware intelligent medical editor better provides enriched information and edit recommendations to a user.

Other sources of data may include the following. Medical ontologies help recognize medical vocabulary and medical reasoning. If a medical word is identified, the system can standardize the mentions, and provide an on screen definition and materials. And based on the user behavior, the system can learn and make our model more accurate. Patient profile and past EMR records, images and test results of a patient allow for personalization of edit recommendation to patient's situation. Clinical guidelines (both global and specific to a hospital or practitioner) help with customization of edit recommendations to standard clinical best-practices. Past patient's EMRs (doctor, departmental, hospital specific) help with customization of edit recommendations to personal/department styles and treatment approaches. User profile and usage logs of user activity allow for personalization of edit recommendations to personal styles and preferences. Current (even if only partially filled) EMR (structured and unstructured) data allows for customization of edit recommendations to the current context and structure of the report being typed. Language models (with support of enriched text) may ensure grammatical and logical consistency and help generate edit-candidates. Synoptic reporting standards 328 such as template suggestions can help identify mandatory items.

Clinical information extraction module 304 can determine and output for a given input (e.g., text string) a statement identification, a subject, predicate, object, source text segment, text start position, confidence score, and consequent statements. The confidence score may be from an artificial intelligence or machine learning algorithm that has provided the analysis, and providing a confidence level that the labelling assigned or determined metadata is accurate. Additional label information may be metadata in some embodiments.

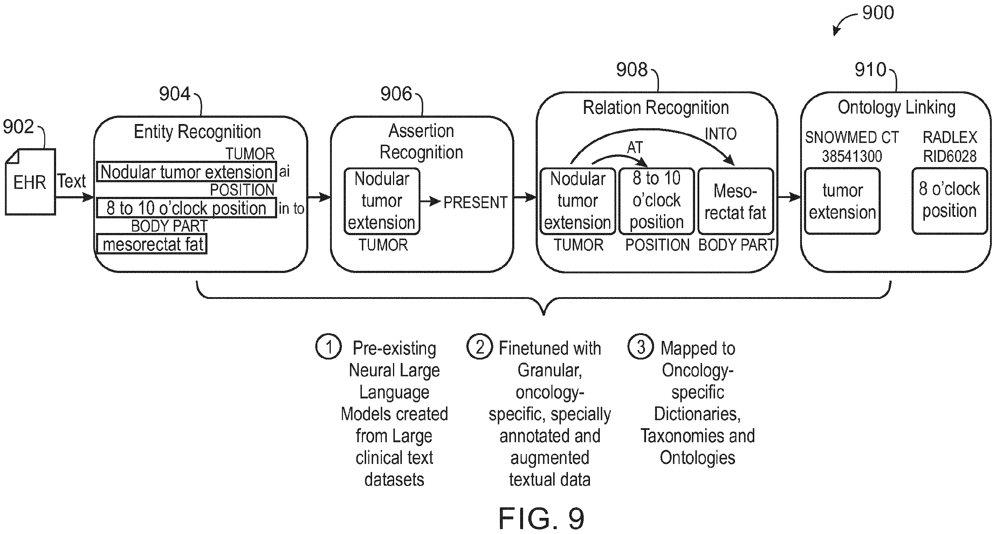

FIG. 9 shows further detail in regards to the clinical information extraction module 900, in an embodiment. These features may be in clinical information extraction module 304. FIG. 9 includes EHR 902, entity recognition 904, assertion recognition 906, relation recognition 908, and ontology linking 910. Clinical information extraction module 900 takes in electronical health record text in EHR 902. Then, pre-existing neural large language models can be created from large clinical text datasets. These may be fine tuned with granular oncology-specific (or other medical expertise), specially annotated and augmented textual data, followed by being mapped to oncology-specific (as an example) dictionaries, taxonomies, and ontologies.

In entity recognition 904, the text is analyzed to recognize entities that may be classified in one of a number of ways. In the example shown, a medical condition (TUMOR) is found in the text, along with a location (POSITION) and part of the body (BODY PART). This then allows the intelligent medical editor system to look through other classifications and related databases for TUMOR, POSITION, and BODY PART, in this example. Further, the system may more clearly analyze information and understand it based on its recognized entity type.

In assertion recognition 906, a subset of text that has a recognized type (TUMOR, in this example) is analyzed and the text indicates the patient has such a tumor. Thus, the tumor exists in the patient and the clinical information extraction module 900 asserts the TUMOR is PRESENT.

In relation recognition 908, the relation between different parts of text that have been recognized as entities are determined. Relationships of time, location, to which patient, by which doctor, which specialty of medicine, which drugs and dosages, and other relationships can be determined by relation recognition 908. In the example of FIG. 9, the TUMOR has been found AT the POSTION and INTO the BODY PART. Thus, a nodular tumor extension (TUMOR) has been found AT 8 to 10 o'clock position (POSITION) and INTO the mesorectat fat (BODY PART). The relationship between data and classification types is very helpful in providing the edit recommendation module with useful context.

Additionally, in ontology linking 910, certain entity types that are related to medical dictionaries, taxonomies and/or ontologies may be further connected with the related content. These may be medical codes, proper medical names, or other agreed upon terminology or verbiage. In the example of FIG. 9, the tumor extension is linked with a standard SNOWMED CT code along with a reference number 38541300, and the position is linked with RADLEX RID6028. SNOMED CT is one of a suite of designated standards for use in U.S. Federal Government systems for the electronic exchange of clinical health information. RADLEX relates to radiology lexicon. RadLex contains individual terms and concepts related to medical imaging that can be used in a variety of applications, including radiology reporting, data mining and research. RadLex Playbook combines RadLex terms, using a consistent syntax, to provide a standard set of exam names and codes. Through the exemplary steps of FIG. 9 illustrating one embodiment, an intelligent medical editor using a clinical information extraction module can gain context about the information coming into the system and build some groundwork understanding that is great support when determining edit recommendations and annotation support.

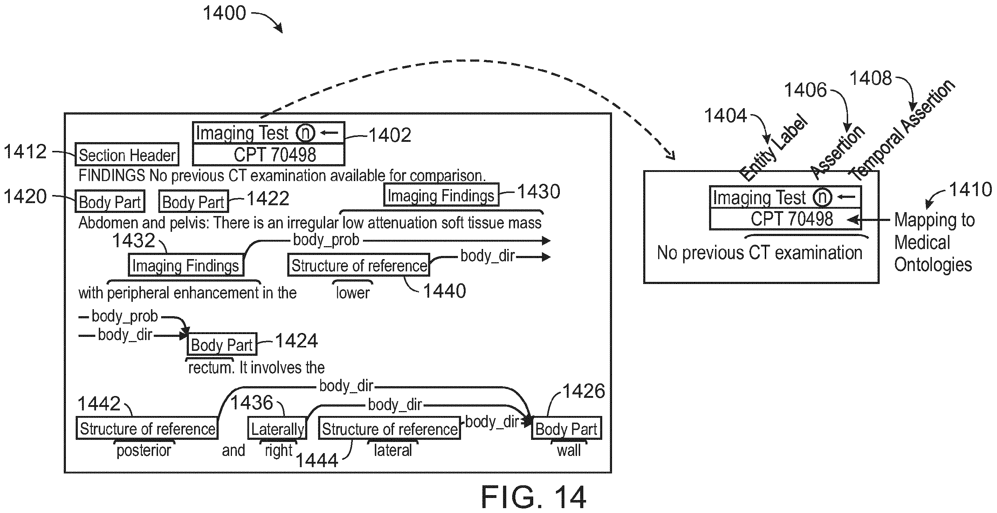

Returning to FIG. 3, enriched information overlay module 306 takes in text data, structured data, and medical data structures 318. Further, enriched information overlay module may take in the output of other modules, such as clinical information extraction module 304. Enriched information overlay module 306 take the input data and finds additional relevant information within the system or from outside the system (e.g., medical dictionaries on the internet, medical guidelines in the hospital intranet, or other external systems). Then, enriched information overlay module 306 can provide additional labels and a deeper level information (if requested by the user) - enriched information - about the content of a medical report. Such labels (also included in enriched information) can be entity labelling for words and phrases, section labelling, identifying the relationship or association between entities, identifying negations, weak assertions, hypotheticals and conditionals, identifying temporal assertions, as well as identifying the ontological concept identification for entities. And this enriched information can be provided to EMR editor 302 and displayed to users for interaction. FIG. 14 shows an example of how an enriched information overlay module can positively impact an intelligent medical editor.

FIG. 14 shows details for an enriched information overlay module, according to an embodiment. FIG. 14 includes enriched overlay output 400, imaging test label 1402, entity label 1404, assertion 1406, temporal assertion 1408, mapping to medical ontologies 1410, section header label 1412, body part label 1420, body part label 1422, body part label 1424, body party label 1426, imaging findings label 1430, imaging findings with body probability pointer 1432, laterally label 1436, structure of reference label with body direction label 1440, structure of reference label 1442, and structure of reference label 1444. Imaging test label 1402 is associated with the CT examination text of the medical report shown in FIG. 14.

Imaging test label 1402 is an example of enriched information overlayed on report text on a display screen. Imaging test label 1402 includes an entity label 1404 (Imaging Test), assertion 1406 (n = none), temporal assertion 1408 ((-). Thus, imaging test label 1402 is showing the text broken down into categories that can be linked and understood with other information. This is also helpful for clicking on each to get more information. As mentioned above, an enriched information overlay module can seek out and/or receive additional information from internal and worldwide sources that can be provided to the user as more information if labels are interacted with. Imaging test label 1402 is indicating in an alternate manner that no previous imaging test of CT type has been done. And the related CPT code for such a CT examination would be CT70498. Current Procedural Terminology (CPT) codes offer doctors and health care professionals a uniform language for coding medical services and procedures. A user could click, in an embodiment, on the CPT code to get listings of other CPT codes and/or more information on the details of this code. This information could be provided by an enriched information overlay module, such as that of enriched information overlay module 306.

Section labeling may be done throughout a data record, medical information, medical records, and medical reports. For example, the section of the medical report shown in FIG. 14 is the FINDINGS section. Thus, a clinical information extraction module has identified the word FINDINGS as a section header and an enriched information overlay module has displayed section header label 1412. Various body parts are also labeled as they come in the report, such as body part label 1420, body part label 1422, body part label 1424, and body party label 1426. As body parts are found, more information may be generated based on how many times the body part appears, the vitalness of the body part, relations of the body part to other listed body parts in the report, and other factors to give additional context to the report and help provide better edit recommendations. Imaging findings label 1430 and imaging findings with body probability pointer 1432 both are findings from an imaging scan by a medical device. And the second has a pointer to a probable body part associated with the imaging findings. This helps the system connect different medical tests to other areas of context for the test, such as the results and/or related body parts. Laterally label 1436 provides information on location and direction of a structure or body party in the report. Structure of reference label with body direction label 1440, structure of reference label 1442, and structure of reference label 1444 give additional information as to where and what part of a body part is being referenced in the report. For example, structure of reference with body part direction label 1440 indicates that it is the lower part of the rectum, which has body part label 1424. The relationship and/or association between labeled entities can be very important when performing machine learning or artificial intelligence analysis as part of an intelligent medical editor. These provide additional enriched information both to the user and the intelligent medical editor. Enriched information from enriched information overlay module is provided both to the user as well as to other modules within an intelligent medical editor.

Returning to FIG. 3, edit recommendation module 308 is an important part of an intelligent medical editor. Edit recommendation module 308 provides edit recommendations to EMR Editor 302 for displaying on a display screen and for a user to interact with using computer input devices. Examples of edit recommendations are detailed throughout the specification and drawings, including with reference to FIGs. 13-17. Edit recommendation module 308 takes in information from throughout the system, such as from contradiction and incompleteness detection module 312, language model finetuning module 314, and user profile and preferences 334.

Edit recommendation module 308 can provide edit recommendations in real-time as a user is editing medical material, such as a medical report or medical record. Thus, edit recommendation module 308 is an element that helps an intelligent medical editor suggest labels/annotations while a healthcare professional is writing a report. One example of edit recommendation is related to the FHIR standard. The FHIR (Fast Healthcare Interoperability Resources) standard defines how healthcare information can be exchanged between different computer systems regardless of how it is stored in those systems. If clinical information extraction module 304 identifies certain parts of the materials may relate to FHIR or certain fields of a report, the edit recommendation module can auto-fill or auto-populate FHIR concepts. The codified FHIR concepts can be parsed from report and edit recommendation module 308 can auto-fill FHIR data of reports, which is a much needed feature both for productivity of doctors as well as enormous benefit to downstream applications.

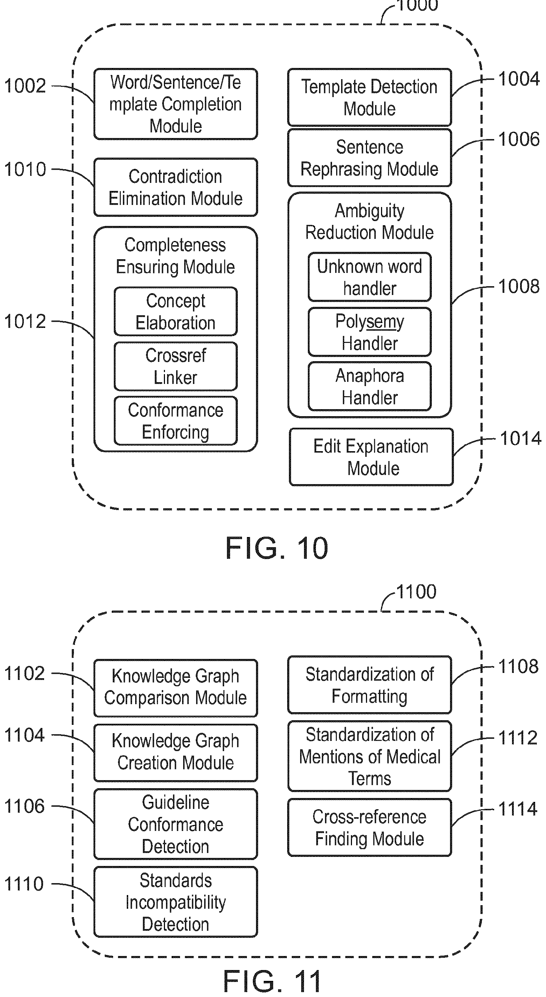

FIG. 10 shows details of an edit recommendation module, according to an embodiment. FIG. 10 includes edit recommendation module 1000, word/sentence/template completion module 1002, template detection module 1004, sentence rephasing module 1006, ambiguity reduction module 1008, contradiction elimination module 1010, completeness ensuring module 1012, and edit explanation module 1014. Edit recommendation module has many types, or categories, of edit recommendations. These may be related to many types of issues discussed herein throughout, including, but not limited to, ambiguity, contradictions, incompleteness, rephasing issues, and auto completion of words, template, thoughts, phrases, paragraphs, or sentences.

Word/sentence/template completion module 1002 is enabled is enabled by large language models (LLM), neural networks, artificial intelligence, generative artificial intelligence, rules engines, and/or machine learning. This may be through language model finetuning module 314 in an embodiment. LLMs have acquired an embodied knowledge about syntax, semantics and ontology inherent in human language. LLMs that help identify edit-candidates may be masked (MLM) or causal LLMs. Causal language modeling is the task of predicting the token following a sequence of tokens.

Further, based on previous histories of reports in a hospital or usage logs 332 of a user or department, patterns can be recognized and/or clinical guidelines may be followed. Word/sentence/template completion module 1002 suggests text and other outputs that may be what the user would prefer and/or intend for the rest of the word, sentence, or template to have in it. This may provide helpful auto-complete suggestions for partially typed words, partially typed sentences, partially typed templates, and various report fields such as in EMRs. The type of report and related template may be detected by the system through template detection module 1004. In certain medical environments there may be a library of relevant templates that template detection module 1004 can be aware of and help to pre-populate.

Sentence rephasing module 1006 analyzes sentences and can suggest rephasing improvements. This may be based on best practices learned from analyzing other reports, based on proper medical phrasing, or based on language issues. Exemplary problems sentence rephrasing module 1006 addresses include a lack of conciseness, lack of clarity, reformatting changes, the need to align to a different template or format, incorrect use of proper medical terminology, and others. Further, if the language of the report is not the first language of the user, sentence rephrasing module 1006 can provide helpful suggestions for the language of the report.

Ambiguity reduction module 1008 analyzes the report and provides suggestions for edit recommendation module 1000 to help improve clarity and reduce ambiguity. Ambiguity reduction module 1008 may address problems such as the presence of an unknown word, incomplete or ambiguous sentences, pronoun issues, anaphora, ellipsis, relative temporal references, polysemous word, acronyms, and abbreviations. Detection and remediation of such problems may be in sub-modules or sub-routines. FIG. 10 shows ambiguity reduction module 1008 including sub-modules of unknown word handler, polysemy handler, and anaphora handler.

Contradiction elimination module 1010 analyzes the report and provides suggestions for edit recommendation module 1000 to remove contradictions. Contradictions may include statements contradicting with other parts of the report, statements contradicting with patient data, statements contradicting with medical ontologies, statements contradicting with clinical guidelines, formatting inconsistencies within the document, formatting contradictions with synoptic standards. For example, if the patient is male and the report says the patient is female, or report says the patient has ovarian cancer, then there may be a contradiction. Sometimes a contradiction may be the result of an error, like a mistype. One part of the report may say a patient weight is 176 pounds and another part of the report may say the patient weight is 17 pounds. Contradiction elimination module 1010 may notice the contradiction and also suggest an edit for display on the screen that the second weight (17) should be updated, potentially to 176.

Completeness ensuring module 1012 analyzes the report and provides suggestions for edit recommendation module 1000 to assist with completeness. Incomplete reports can cause medical issues and waste time. Incompleteness issues solved by completeness ensuring module may include a lack of cross-reference links (e.g., to prior reports, images or medical reference data sources), incompleteness in complaint, diagnosis or treatment history, broader concept in place of more a granular concept, and missing aspects compared to reporting standards. Many medical professionals are under a time crunch and may be tempted to not fully complete orders, reports, and records. By providing specific edits and suggested text, they may feel less burden as they work to complete the word, thus improving patient care and speeding up medical workflows. One type of suggestion that may be output on a user screen is to automatically follow up with another hospital or physician. If the patient medical record is incomplete, the system can automatically generate a communication to the entities likely to have the needed information. Then an edit recommendation can be shown on the screen such as "contact Dr. Faber for a copy of the imaging scan?". If the user clicks on the recommendation, a draft email or other communication type can be shown on screen already filled out and ready to be edited and/or sent. This can help with completeness of records without burdening medical professionals. Completeness ensuring module 1012 may include the sub-modules of concept elaboration (adding more text when a concept is not fully elaborated), cross reference linker (if multiple sections of a report can be cross-referenced for better understanding a link is provided), and conformance enforcing.

Edit explanation module 1014 provides additional information and context to why a certain edit was recommended. A user using an intelligent medical editor may not understand why a suggestion was recommended or how the recommendation was generated. By clicking on the recommendation (or provided automatically without a user input prompt), the intelligent medical editor can provide additional information provided by edit explanation module 1014. Such explanations may explain why a cross reference was made and may provide a link to the relevant patient data model and clinical guidelines. The modules shown in FIG. 10 are not an exhaustive listing, and other features herein throughout may be provided by edit recommendation module 1000.

Returning to FIG. 3, usage and feedback logging module 310 is vital for processing the requests of the user, as well as training the system for future edit suggestions. How each user interacts with the system is received from EMR editor 302 as user decisions and feedback. Usage and feedback logging module 310 records the user decisions and feedback, provide them to usage logs 332 and/or user profile and preferences 334. Both the explicit requests from the user may be logged, as well as the individual usage pattern (clicks, touches, visual contact, and any other type of input) of the user. There may be pop up windows or other on-screen or voice feedback to and from the user asking how the experience was or improvement suggestions on specific edit recommendations. These are especially valuable when a user does not take the recommended edit. The edit suggestions may have different types of feedback, such as there is an error, a miss, or the data provided was not granular enough, or too granular.

Contradiction and incompleteness module 312 may be its own module as shown in FIG. 3 in an embodiment, or may be a part of edit recommendation module as shown in FIG. 10, in an embodiment. See details for contraction elimination module 1010 and completeness ensuring module 1012 noted above. Contradiction and incompleteness module 312 may detect discrepancies between what is being written and what is available from a patient's history, clinical care, and medical ontologies.

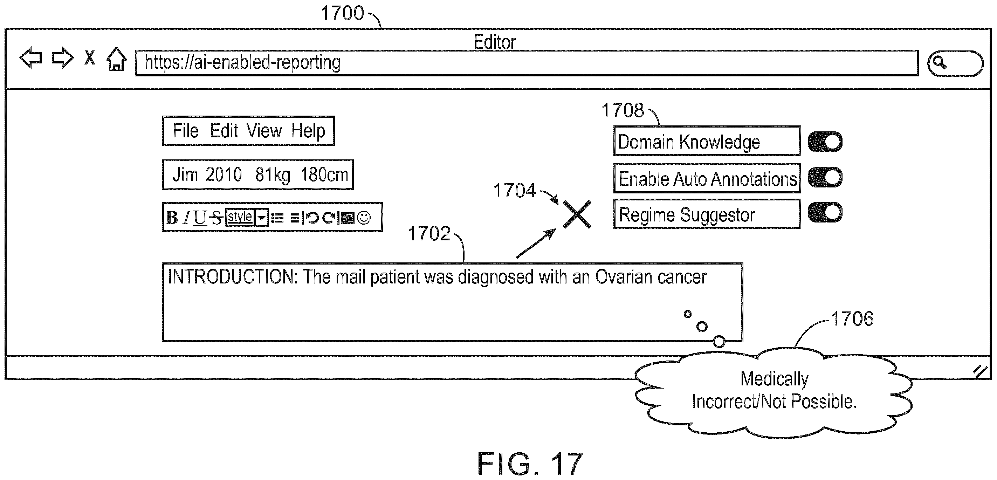

FIG. 17 shows a user interface with medical corrections in a digital document, according to an embodiment. A contradiction and incompleteness module as identified a contradiction 1704 with the introduction of a medical report 1702 in user interface 1700 of an intelligent medical editor. A visual icon may be displayed. A pop-up window 1706 or side-bar window may be displayed to explain the detected contradiction or error. And a suggestion may be made to the user for automatic correction. In the example of FIG. 17, the intelligent medical editor may suggest changing the word `male' to `female' in a report. FIG. 17 also shows toggle switches related to features of an intelligent medical editor. These may include domain knowledge option 1708, enable auto annotations option, and regime suggestion option. Thus, a user has control over the edit recommendations and can tweak features based on their preference. In addition, the contradiction and incompleteness module may detect that the word 'male' is incorrectly `mail' in the report, and either auto correct or provide a user prompt with the edit suggestion.

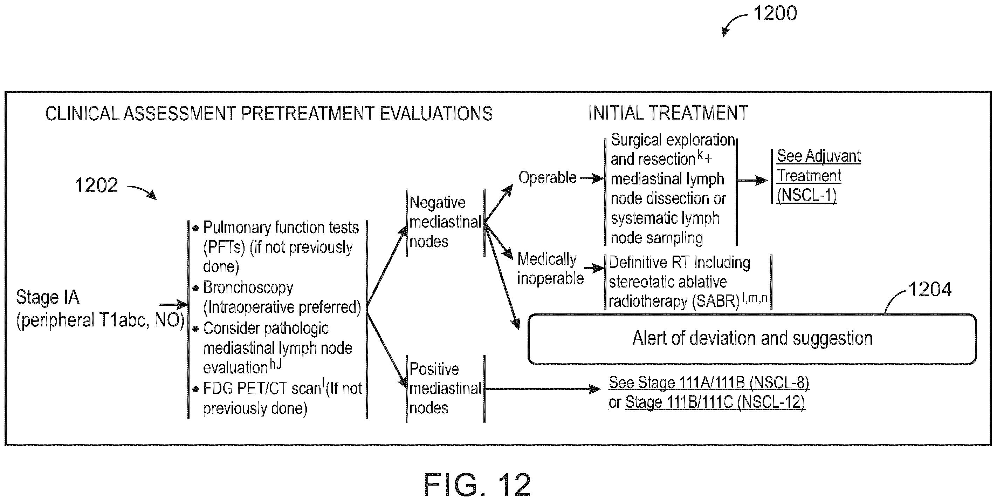

FIG. 11 shows a contradiction and incompleteness detection module, according to an embodiment. Such a module may perform factual contradiction and incompleteness detection using patient data model, clinical guidelines and medical ontologies. FIG. 11, in addition to the discussion in FIG. 3 and FIG. 10, gives additional technical detail related this module. FIG. 11 includes contradiction and incompleteness detection module 1100, knowledge graph comparison module 1102, knowledge graph creation module 1104, guideline conformance detection module 1106 (further discussed in relation to FIG. 12), standardization of formatting module 1108, standards incompatibility detection module 1110, standardization of mentions of medical terms module 1112, cross-reference finding module 1114.

Knowledge graph comparison module 1102 may take in the output of clinical information extraction module and include many fields, such as statement ID, subject, predicate, object, source text segment, text start position, confidence score, and consequent statements. A knowledge graph in the system may be compared with a knowledge graph for the specific record being edited to find contradictions and incompleteness. Such knowledge graphs may be created by knowledge graph creation module 1104, which may take in data about the patient (such as structured data including statement ID, date, subject, predicate, object pointers to the source information, patient journey stage, confidence score, and consequent statements). A knowledge graph specific to a patient may also be thought of as a patient data model, in one embodiment. Knowledge graphs may be generated and compared using semantic networks. A semantic network is a knowledge structure that depicts how concepts are related to one another and illustrates how they interconnect. Semantic networks use artificial intelligence (AI) programming to mine data, connect concepts and call attention to relationships. tagging, using the CPU, parts of speech in the text; recognizing, using the CPU, known words in the text; and creating, using the CPU, a semantic network, the semantic network including at least one of the recognized known words and at least one relationship with at least one semantic concept associated with at least one of the recognized known words; and wherein the output pertains to a probability of a particular occurrence.

Incompleteness detection and report structure recommendations may be provided by standardization of formatting module 1108 using synoptic reporting standards, such as synoptic reporting standards 328. Standardization of mentions of medical terms module 1112 reviews the usage of medical terms and provides suggested edits to align the common usage, spelling, and abbreviation of medical terms in the report. Standards incompatibility detection module 1110 compares the report with medical industry standards, such as billing codes, cariology guidelines, medical rules, and hospital requirements. Cross-reference finding module 1114 detects if there should be a cross reference between information items and provides an edit suggestion to add the cross reference.

Returning to FIG. 3, language model finetuning module 314 works with clinical language models 330, industry data/standards, and usage logs 332 to update and fine tune a language model. This may be LLM as discussed herein, and may include generative artificial intelligence capabilities to continually update and fine tune the language model. Language model finetuning module 314 provides edit recommendation module 308 with specific language to use in edit recommendations based on the type of report, user involved, context of the text, and other factors such as medical ontologies 332, medical data structures 318, patient profile and past Dx/Tx data 324, clinical guidelines 326, synoptic reporting standards 328, and user profile and preferences 334. Language model finetuning module 314 allows the intelligent medical editor system to re-align reports to different templates using Large Language Models. Further, language model finetuning module 314 may be connected with and share bi-directional information with NLU model updation module 316 as both modules deal with natural language.

Medical data structures 318 may be of many types depending on the medical EMR company, IT suppliers, government regulations around medical data, what fields may be needed for certain disease types, varying report record types depending on medical specialty, database requirements, and other factors.

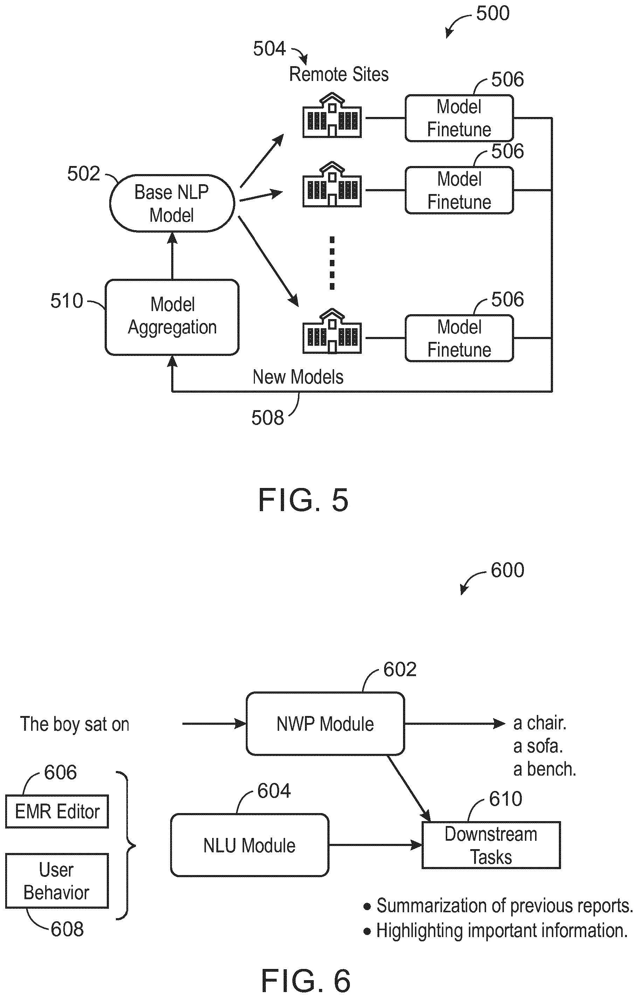

NLU model updation module 316 receives usage logs 332 as well as Federated NLU models. NLU model updation module 316 provides updated NLU models to NLU models 320. These NLU (natural language understanding) models may include NLP (natural language processing), NLG (natural language generation) and NWP (natural word processing) models as well. These will be further explained in connection with FIGS. 5, 6, 8A, 8B, and herein throughout. NLU models 320 are provided throughout an intelligent medical editor as many parts of the system need natural language processing, generation, and understanding, such as to clinical information extraction module 304, enriched information overlay module 306, edit recommendation module 308, usage and feedback logging module 310, contradiction and incompleteness detection module 312, and language model finetuning module 314.

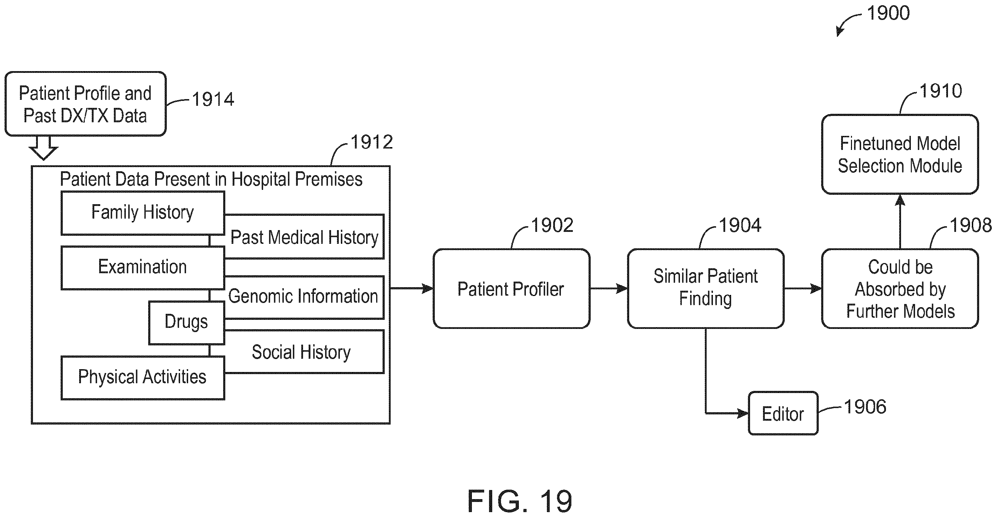

Patient profile and past Dx/Tx data 324 is a collection of all the patient data throughout the system. The more accurate data about a patient, the more insights and recommendations an intelligent medical editor can make. Patient profile data may be from an EMR, medical reports, medical imaging scans, medical notes, communication from a patient, and more. Patient profile data can include their allergies, problems, history, and medications. FIG. 19 discusses more about patient data present in hospital premises and using patient profile data to determine insights and recommendations.

User profile and preferences 334 stores and analyzes information about past, current, and future users of EMR editor 302. Each user may have a different role (such as nurse, primary care physician, radiologist), a different aptitude for using software technologies, a different knowledge of medical terminology, a different knowledge of medical standards, and the like. User profile and preferences 334 takes information in from usage and feedback logging module 310 and can provide output to any module, but in specific to edit recommendation module 308. An edit related to a report may be different based on the user involved at times. For example, one user consistently uses abbreviations vs. another user always types out the full word. Or if one user has a pattern of using a certain medication to treat the same patient disease, it can be suggested as a recommendation in the future. Thus, pattern recognition and artificial intelligence learning is helpful for user profile and preferences 334, and the module may have such technology built in.

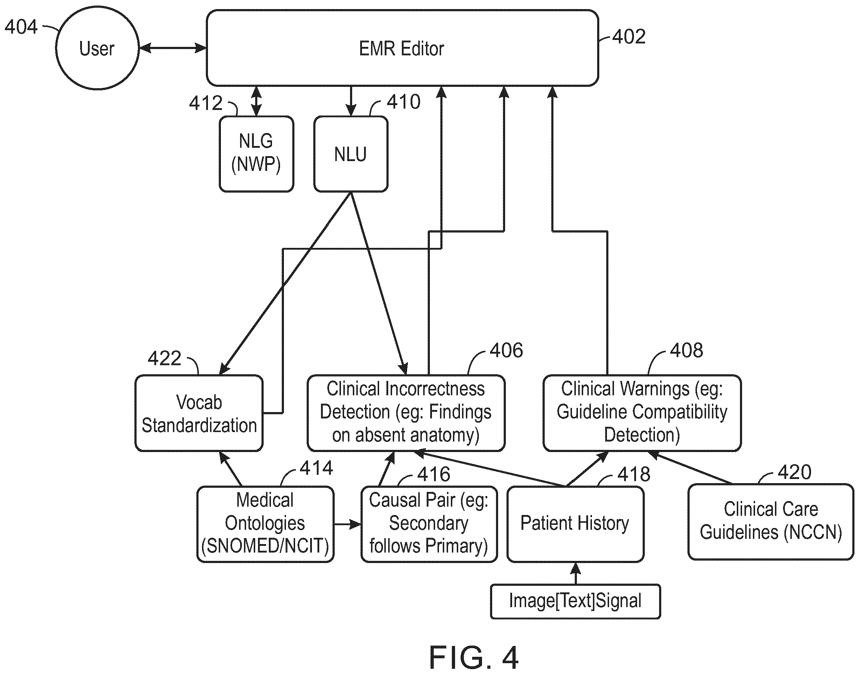

FIG. 4 shows a flow diagram for an intelligent medical editor, according to an embodiment. FIG. 400 includes EMR editor 402, user 404, clinical incorrectness detection 406, clinical warnings 408, natural language understanding 410, natural language generation and natural word processing (NWP) - NLG 412, medical ontologies 414, causal pair 416, patient history 418, clinical care guidelines 420, and vocabulary (vocab) standardization 422.

EMR editor 402 interacts with user 404 through computer input and output systems. User 404 uses EMR editor 402 as an intelligent medical editor to generate, edit, and improve medical materials, such as radiology reports, electronic medical records, and prescription orders. Clinical incorrectness detection 406 and clinical warnings 408 may be modules that receive medication information from input modules such as medical ontologies 414, causal pair 416, patient history 418, clinical care guidelines 420. In the embodiment of FIG. 4, EMR editor 402 may have an edit recommendation module within it. EMR editor 402 can receive warnings and incorrectness detection and determine edit suggestions with the help of language interaction enabled in the system. EMR editor 402 provides displayed edit suggestions to a user for the user to select and interact with, as shown in FIG. 13, for example.

Clinical incorrectness detection 406 and clinical warnings 408 may have analysis code with software algorithms and/or artificial intelligence models in various embodiments. Clinical incorrectness detection 406 leverages medical ontologies 414 and causal pair 416 to find patterns and relationships between information in patient history 418 and medical conditions that are being evaluated in a patient. Clinical incorrectness detection 406 then detects if there is a clinical incorrectness of the information currently in a report and what is medically possible, based on the causal pair 416 and patient history 418. Patient history 418 may receive information signals about a patient in image and/or text forms. An example of a detection of clinical incorrectness is if a patient has no left leg and an imaging report says that the left knee is broken. Causal pairs 416 would detect that the body part was removed during a past surgery (cause), and therefore the current report can't have findings based on that body part. Or if a patient has ovarian cysts but the patient does not have ovaries. Finding such incorrectness can save lives, time, and the reputation of the medical professional.

Clinical warnings 408 can provide detected warnings to EMR editor 402 for displaying on a user display. Such warnings may also provide suggested edits and/or changes, in various embodiments. Warnings can be popped up if a treatment decision is taken which is not present in any of the treatment options suggested by care guidelines. Such guidelines may include the National Comprehensive Cancer Network (NCCN) Guidelines for cancer. The possible treatment options can be found by traversing through the guidelines using the patient history.

Language interaction is enabled through NLG 412, NLU 410, and vocab standardization 422. These are further discussed, among other places, with respect to FIGs. 3, 5, 6, 8A, and 8B. NLG 412 generates natural language, and includes a next word prediction module to suggest next words while writing reports. NLG 412 may perform the acts of content determination, document structuring, aggregation, lexical choice, referring expression generation, and/or realization. NLU 310 takes in the input report and patient history and generates a natural language understanding of the content. NLU includes rearranging unstructured data so that the module may "understand" and analyze the data. This natural language understanding thus can be compared against medical ontologies 414 and clinical care guidelines 420 as examples. Medical ontologies may include tables such preferred name (such as Cisplatin/Epirubicin), synonyms (such as Cisplatin/Epirubicin, Cisplatin-e therapeutic implant, CDDP-e TI, and CDDP/EPI), IDs of the medical ontology (such as a web address of a medical body or organization and a link to the specific page), and code (such as C11555). Vocab standardization can take in the medical ontology information, including the synonyms. All the mentions of a medical phrase including short forms or synonyms will be mapped to the standard index from ontologies. For example: non-small cell lung cancer ~ NSCLC ~ C2926. This helps in standardizing reports irrespective of writing styles. Further if a user editing a report calls the same thing three different ways (such as through abbreviations or shorthand or slang), vocab standardization 412 can suggest consistent naming throughout a record or report related to the thing.

FIG. 5 shows a natural language processing (NLP) model update module, according to an embodiment. FIG. 5 includes networked system 500, base NLP model 502, remote sites 504, model finetune 506, new models 508, and model aggregation 510. Base NLP model 502 is an NLP model that is trained in generic enough tasks to do downstream applications. A goal of networked system 500 is to provide the benefits of long-term improvement of NLP due to higher volume of manually annotated data. This also may help improve downstream clinical applications.

After a base NLP model 502 is provided to medical remote sites 504, medical staff at remote sites use the model in their intelligent medical editors. Through the process of choosing edit suggestions, corrections, reacting to information, and receiving feedback, the model is fine tuned for that specific remote site. The finetuned model 506 then is a new model 508 that can be provided back to the originator of NLP model 502. Using model aggregation 510 technology, the base NLP model 502 can be improved for the system as a whole, not just the individual remote site 504. Model aggregation 510 takes in new models 508 based on finetuned models 506 from a plurality of remote sites 504. NLP model 502 may be a large language model in an embodiment.

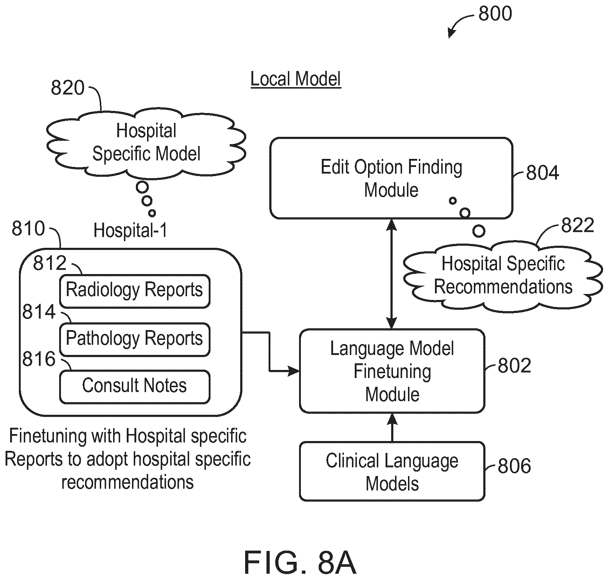

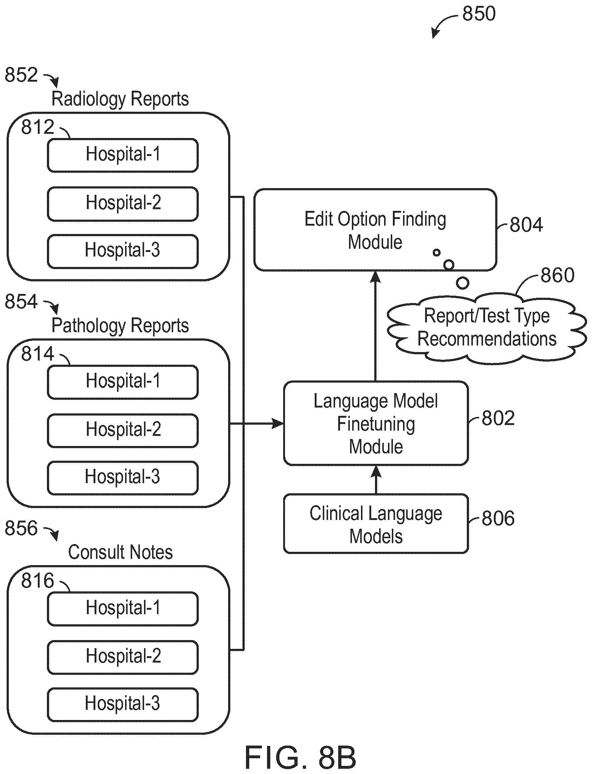

Networked system 500 in FIG. shows how NLP models may be improved through crowdsourcing of feedback and improvements. This allows for getting clinicians (oncologists, nurses, and primary care physicians) to participate in crowdsourcing of technology. Ensuring that the AI and NLP models are performing adequately to meet clinical standards is crucial before integrating them with a critical clinical system like EMR or other medical reports. High volume, variety and veracity of manual feedback is essential for validating and continuously improving the AI and NLP models. The two roadblocks which hinder such a collection of feedback are the high time and cost of high-quality manual feedback, and the data privacy requirements which result in administrative and data anonymization overheads. The systems herein provide (1) creating a low-intrusion way for clinicians to provide in-place feedback to AI and NLP as well as by creating a federated method to improve the AI and NLP models. This may eliminate the need to share patient data outside of the organization/legal boundaries, thereby enabling wide crowdsourcing of training/validation data for such models. Thus, improvements may be done in a federated way without compromising the patient privacy. These benefits and features are also relevant to the systems shown in FIG. 8A and 8B.

That said, each remote site 504 still may have a finetuned model 506 particular to its site, its guidelines, its people, jurisdictional cultural or legal requirements, and the relevant tastes of that site. The system can customize the usage of an intelligent medical editor to the taste of specific users, specific departments, and specific locations.

FIG. 6 shows a natural language generation (NLG) module with a natural word prediction (NWP) module, according to an embodiment. FIG. 6 includes natural language system 600, NWP module 602, NLU module 604, EMR editor 606, user behavior 608, and downstream tasks 610. Downstream tasks 610 may be many in a medical environment, and include the use of such outputs in an intelligent medical editor such as EMR editor 606. Downstream tasks may include summarization of previous reports as well as highlighting important information.

NWP module 602 has the task of predicting an upcoming word(s) (or providing multiple suggestions) based on the input string of text, images, or other input content. The predicted future words are provided to downstream tasks 610. In the simplified example of FIG. 6, NWP module 602 takes in the input of "The boy sat on", and predicts the next words as possibly "a chair", "a sofa", or "a bench".

EMR editor 606 may provide input for natural language system 600 from the content of the EMR or other medical materials. In addition, a current user behavior 608 provides real time input for natural language system 600. User behavior 608 may influence NLU module 604 and NWP module 602 because each user may have a different language style. Because of the varying styles, which may include education levels and adherence to proper terminology, natural language system 600 may be able to incorporate the styles of users and provide that in the edit recommendations to other users. For example, if there is a head of a radiology department who does their work excellently or wants consistent work across their employees, the system may use NLU module 604 to understand the user behavior 608 of the key user and then provide the staff members of that department related edit recommendations in their medical materials. Further, if government officials or regulators expect medical reports and materials to be written in certain standard ways, this also can be fed into NLU module 604 and NWP 602 to improve edit recommendations for users. In an embodiment, when edit recommendations come from certain sources or based on certain inputs, they can be color coded or marked in certain ways. For example, if an edit recommendation has three options, a user-customized edit recommendation may be in one color or marking, a head of department edit recommendation may be in a second color or marking, and a government edit recommendation may be in a third color oar marking. Providing users a bit of context and information as to the origin of certain edit recommendations in an intelligent medical editor can be helpful their adoption and proficient use of such a tool. It may also help shape behavior.

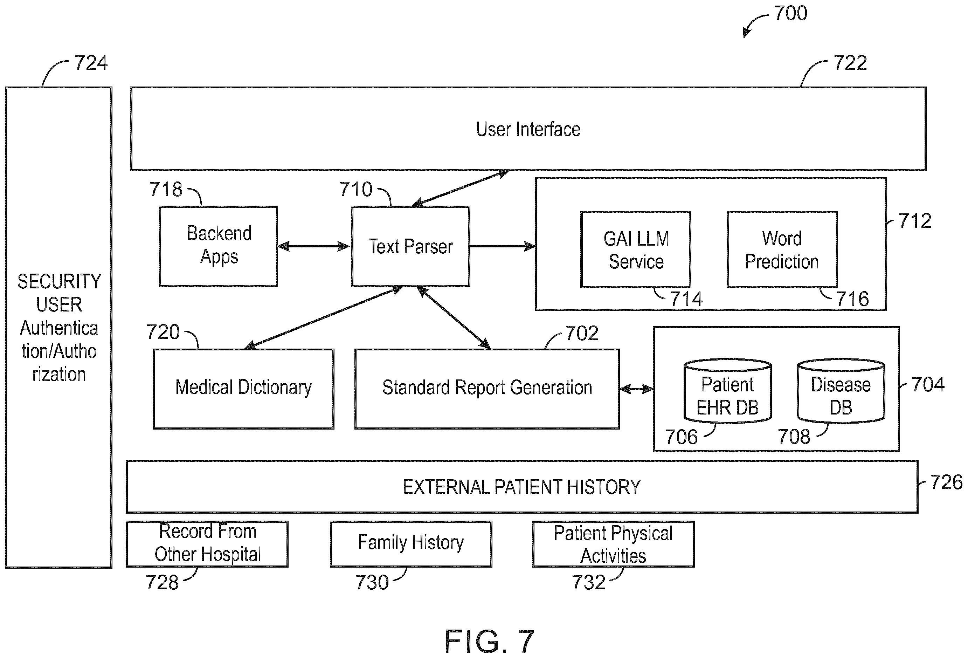

FIG. 7 shows a block diagram supporting natural report generation, according to an embodiment. FIG. 7 includes system 700, standard report generation 702, medical information 704, patient EHR DB (database) 706, disease DB 708, text parser 710, language system 712, generative artificial intelligence & large language model service 714 (GAI LLM service), word prediction 716, backend apps (applications) 718, medical dictionary 720, user interface 722, security layer 724, patient history 726, record from other hospital 728, family history 730, and patient physical activities 732.

An intelligent medical editor may use report generation capabilities of a system like system 700 which includes standard report generation 702. Standard reporting can be done for different types of diagnosis and treatment. Standard report generation 702 may take in information from text parser that has come to text parser from a variety of sources, such as patient history 726, user input through user interface 722, backend apps 718, and medical information 704. Standard report generation takes report templates or previous report examples and generates a new report. Then it prepopulates areas that it knows are related (such as patient names and other factual data from the record). It also, in an intelligent medical editor descripted throughout, can provide edit recommendations and suggestions for other parts of the report that are not fully known or may need a medical professional to make the selection. Thus, a user can interact with a report that has a large portion of it already complete. And standard report generation 706 generate the reports with prepopulated information and edit recommendations based on previous user reports, other reports, medical standards, and third party reports. This allows for the leveraging of best practices. Further, a large amount of time can be saved through such a system. Text parser 710 may use GAI and LLM service 714 as well as word prediction 716 to better provide standard report generation 702 with factual information for prepopulation as well as information to improve edit recommendations. Text parser 710 has direct access to medical dictionaries 720 to help it understand obscure or uncommon medical terminology.

Patient history 726 may include internal and external information. Internal information may include patient EHR records from patient EHR DB 706. Patient history 726 may also include records from other hospital 728, family history 730, and patient physical activities 732. These may be from other devices such as smartphones, smartwatches, activity trackers, medical devices at home or in a medical environment, family history in EHR records of family members, and other locations.

Backend apps 718 may be specific software applications to the disease, country, hospital, care pathway, or other targeted group. Backend apps 718 may be provided by specific vendors and may help with information related to specific reports, such as government required reports that certain vendors have expertise in generating and/or pre-populating. This allows for more efficient and accurate report generation. Further backend apps 718 may be apps related to natural language processing or interaction to help text parser to an improved job handling certain types of information. Security authorization layer 724 allows for proper security and authorization to not allow important user and patient information to leak.

FIG. 8A shows a local model for language fine tuning, according to an embodiment. FIG. 8A includes finetuning system 800, language fine tuning module 802, edit option finding module 804, clinical language models 806, hospital-1 810, radiology reports 812, pathology reports 814, consult notes 816, hospital specific model 820, and hospital specific recommendations 822. Hospitals may have specific recommendations 822 provided to its users by edit option finding modules 804, also called edit recommendation modules, of intelligent medical editors. Hospital specific recommendations 822 may be from hospital guidelines or rules. These recommendations may also be based on detected best practices from medical materials of hospital-1 810 such as radiology reports 812, pathology reports 814, and/or consult notes 816. Thus, hospital specific models 820 can be generated for language and edit recommendations. Clinical models 806 can provide publicly available language models trained on large corpus of medical documents which can then be specialized by language model finetuning module 802 and/or edit option finding module 804 to perform hospital specific downstream tasks.

Language model finetuning module 802 receives clinical language models 806 along with medical materials specific to hospital-1 810, such as radiology reports 812, pathology reports 814, and/or consult notes 816. Through this, language model finetuning module 802 can finetune its language model with hospital specific reports allowing for the adoption of hospital specific recommendations. For example, hospital-1 may be in a location where certain terminology or language is used that is different than other hospitals, and certain vernacular is more common. Another example is if a certain hospital requires a specific radiology report section in its reports that is not required by other hospitals (for insurance or government billing in an example). As another example, models may need to be finetuned to suit for specific application to achieve required outcome (such as clinical word classification, relation assertion, word/sentence classification, etcetera.) Language model finetuning module 802 can provide a hospital specific model to edit option finding module 804, allowing edit option finding module 804 to suggest edits that are best suited to the hospital in question. This may also be done at more specific levels as well, such as per department or individual user. These aspects of the systems and methods can aid EMR and reporting tasks such as document summarization, similar document and finding key information to prepopulate a medical report.

FIG. 8B shows details for a language fine tuning module, according to an embodiment. FIG. 8B includes finetuning ecosystem 850, radiology reports 852 which includes radiology reports 812 from hospital-1 and other hospitals, pathology reports 854 which includes pathology reports 814 from hospital-1 and other hospitals, consult notes 856 which includes consult notes 816 from hospital-1 and other hospitals, language finetuning module 802, edit option finding module 804, clinical language models 806, and report/test type recommendations 860. FIG. 8B shows the value of distributed and/or crowdsourced information to help improve language models and edit recommendations. This allows clinicians, oncologists, nurses, assistance, physicians, and others in the medical system to all provide helpful feedback to the system for improving language models and edit recommendations in intelligent medical editors. Further, a model can be fine-tuned not just on the users of the system or requirements from the hospital or government, but also based on the patient. For example, fine tuning may be based on the timeline history of patients, type of disease, type of treatment, equipment use, medication dosages, and other factors along a patient j ourney. To effectively aid the aid the recommendations, multiple models could be aggregated, and recommendations could be based on the certain criterion / domain specific rules.

In FIG. 8B, finetuning ecosystem 850 exists to collect similar types of medical materials from different medical institutions, such as hospital-1, hospital-2, and hospital-3. Through this information, language model finetuning module can create models based on trends across geographies, medical systems, and different sizes of medical systems, all while developing a model specific to the type of medical materials involved. Thus, language models and edit recommendations can be specific and predictive to the report/test type, as shown in report/test type recommendations 860. For example, terminology in an oncology care pathway may be different from a cardiology care pathway.

FIG. 12 shows a clinical care guidelines deviation flow, according to an embodiment. FIG. 12 also relates to how clinical guidelines 326 may interact in the system, including with contradiction and incompleteness detection module 312. FIG. 12 includes clinical guidelines workflow 1200, clinical guidelines 1202, and deviation 1204. Clinical guidelines 1202 in this example are clinical assessment pretreatment evaluations which may suggest initial treatments. Certain tests may be run for a patient and based on the results of the test, certain treatments are recommended and/or required. If a user selects a treatment, medical examination, and/or medication that is non-standard in clinical guidelines 326 this may be flagged as a deviation 1204. An intelligent medical editor may display an alert of deviation and suggest alternative recommendations. One example of this with relation to FIG. 13 is contradiction notice 1322. A recommendation may be that 'the guidelines have suggested this initial treatment'. Further, if different countries have different guidelines, an intelligent medical editor can provide the correct guideline for this location/hospital, and also provide suggestions of how others may be doing in other hospitals/around the world.

Such a system can improve care and reduce multiple decisions that can lead to variations in medical care. Thus, such a system can save costs and provide better care to patients. Unwarranted deviations in care leads to suboptimal care, cost and affects quality of life. Clinical standardization remains on the top of the cost saving opportunities for CFOs while maintaining quality. Cancer guidelines and pathways guide physicians to render care that's clinically optimal and cost efficient through data and flowcharts. High complexity and multiple treatment possibilities of the cancer disease makes this very challenging to adhere to and leads to widespread deviations. An example for lung cancer guidelines shown in FIG. 12 illustrates the numerous decision points and data needed to traverse the guideline to the relevant point.

If a deviation from a guideline is approved by the user, the system can save the deviations for reporting and analysis. And then in the future, the system can check if a current deviation is consistent with deviations in the past for the same type of patient/situation, and provide a percentage confidence of previous deviations. An intelligent medical editor can also suggest what have the approved deviations in the past on a similar patient. Further, the system can provide statistics related to deviations, such as how many deviations are there for the clinician. And in some circumstances, if there is consensus across a medical field, the guidelines may be changed based on collective deviations that are good for healthcare.

FIG. 13 shows a user interface with suggested edits for a digital document, according to an embodiment. FIG. 13 includes medical editor 1300, user interface 1302, event date recommendation 1304, internal conflict recommendation 1306, date recommendation 1308, missing information recommendation 1310, formatting recommendation 1312, spelling recommendation 1314, acronym expansion recommendation 1316, image source recommendation 1318, ontology code linking recommendation 1320, contradiction notice 1322, and diagnosis recommendation 1324. User interface 1302 shows a medical report that already has some content. This content may have been typed in, prepopulated by the intelligent medical editor, or a combination of the two. The report has headings for different sections of the report that are shown in capital letters on the left. A system, such as that of FIG. 2, reviews the material in the report and can provide alerts, notices, and recommendations to help improve the content of the report.

These alerts, notices, and recommendations may be provided in different ways. FIG. 13 shows the alerts, notices, and recommendations as overlaid above the relevant section of the report. Alternatively, these may be little alert bubbles or icons that are clicked on for more information. Alternatively, these may be in a side bar or top bar of a user interface window. Alternatively, these may be through an interactive chatbot or conversation style interaction. These may be supported by generative artificial intelligence and large language models. There may be other ways of alerting and interacting such as vibration, touch, voice, and other audio inputs. The alerts, notices, and recommendations may also have a feedback button or icon. This allows a user to indicate errors, issues, suggestions, or positive feedback on the alert, notice, or recommendation. The system can use this to improve its language models and edit recommendations.

Event date recommendation 1304, is an example of adding additional information to the report that helps provide full detail. In the example, medical editor 1300 recommends adding "on Aug 23^{rd}, 2022" to the report referring to the day the patient presented their medical complaints. This improves precision via event date recommendation 1304 using a patient's past data. This may be done through a patient profile as further discussed with reference to FIG. 19. A medical editor may detect a reference to a past event in a report based on a language model that looks at past tense words, tracks the patient j ourney, and/or certain fields in an EMR, as examples. If more information is available about the past event, an edit recommendation module may make a recommendation like event date recommendation 1304. More information about the past visit could be suggested, and both a short recommendation and long recommendation may be provided. And based on the interactions of the users (which selections are made most often), the intelligent medical editor can prioritize the recommendations user's most prefer. Date recommendation 1308 also suggests adding a date related to a recent medical test, in this case a mammogram.

Internal conflict recommendation 1306 may use the rest of the report or other patient profile information to detect conflicts. In this example, the report in the fourth line states the "left breast." And thus, in the third line where it says, "right breast", an intelligent medical editor (for example through a contradiction and incompleteness detection module 312) can detect internal inconsistencies and ask a user for clarification. If a user clicks on the internal conflict recommendation 1306 which is "left?" in this case, the intelligent medical editor can automatically change the word right into left. Another internal conflict example is the wrong date listed in a report, such as those related to event date recommendations.

Missing information recommendation 1310 may provide recommendations for additions to medical materials. This would be helpful information that may be added to the report but currently does not exist. In the example of FIG. 13, a STAGE field of a medical report only says, "Stage 1." Stage refers to the extent of cancer, such as how large the tumor is and if it has spread. Missing information recommendation 1310 suggest it say TNM STAGE: T1, N0, and M0. The T refers to the size and extent of the main tumor. The main tumor is usually called the primary tumor. The N refers to the number of nearby lymph nodes that have cancer. The M refers to whether the cancer has metastasized. This means that the cancer has spread from the primary tumor to other parts of the body. Thus, with the addition of the missing information recommended, the report is more accurate, clear, and helpful to an oncologist reviewing the cancer situation.

Formatting recommendation 1312 highlights ways in which formatting can be improved in a medical report. For example, certain report types are required or recommended to be in certain formats. In other examples the language model can detect that a report has a custom format and edit recommendation module can recommend edits related to maintaining the custom format. In the example of FIG. 13, all of the other report headings are in capital letters, and thus the medical editor 1300 recommends changing "Recommendations" to "RECOMMENDATIONS." This improves readability by recommending formatting variations. Other types of formatting, spelling, and grammar edits may also be recommended using related modules. Certain types of spelling may be specific to medical ontologies and medical lexicography. Spelling recommendation 1314 notes a misspelling of a specific drug (not in common dictionaries) and provides a corrected spelling recommendation. Having medical specific dictionaries, guidelines, and codes is useful. In addition, certain types of UB-04 and CPT codes could be in EMR records and intelligent medical editor can detect inaccuracies, incorrectness, and misnaming issues. The Uniform Billing Form, known either as the UB-04 or CMS 1450, is a key player in the healthcare billing process. Further, if a report has a synoptic reporting standard, the system can identify parts of a report that are not following the synoptic reporting standard. Synoptic reports present information as discrete data elements and associated responses.

Acronym expansion recommendation 1316 helps standard communication in medical reports. When a certain user uses a non-common acronym (or one that is better at complete words), an edit recommendation module can suggest that an acronym be fully stated. These may come from medical dictionaries or medical ontologies. This is helpful and more precise if a report or record will be viewed by patients or other non-medical experts who may not know all the acronyms that medical experts may use. In the example of FIG. 13, the report has the phrase "UOQ of the left breast." Acronym expansion recommendation 1316 recommends changing "UOQ" to "upper outer quadrant."

Image source recommendation 1318 can provide additional source information about content. This may be the internet source of content with a hyperlink, it may be the source location in an imaging database such as a PACS - Picture Archiving and Communication Service, or a source location in a patient profile or medical record. By adding a link or other source information, a person reading the report is better equipped to look into the details of the situation and make better decisions in a faster way. In the example of FIG. 13, the mammogram image of the left breast showed a mass in the upper outer quadrant. Image source recommendation 1318 recommends adding in the exact series and images related to the mammogram. Image source recommendation 1318 may also have a clickable link for a viewer to see the exact images. This improves completeness via image source recommendation using a patient's past radiology image data.

Ontology code linking recommendation 1320 uses medical ontologies to improve precision in a medical report. In the example of FIG. 13, medical editor 1300 recommends adding ICD codes 147.9 and 174.4 to the report. International Classification of Diseases (ICD) is a system used by physicians to classify and code all diagnoses, symptoms and procedures for claims processing. For example, ICD-9 code 174.9 for malignant neoplasm of breast (female) unspecified site is a medical classification as listed by WHO under the range. This is valuable to add to the report of FIG. 13 because it is in the specific section of the report of diagnosis. So having all the proper terminology and codes for the diagnosis is valuable.

Contradiction notice 1322 notices a contradiction of a recommended treatment compared to clinical guidelines. This is discussed further with respect to FIG. 12, and improves precision via finding contradictions with current medical knowledge using clinical guidelines. In the example of FIG. 13, medical editor 1300 notices that the treatment recommended contradicts with the NCCN (National Comprehensive Cancer Network) BINV-1 guidelines around breast cancer treatments, and recommends adjustments to the recommended treatment plan. In some embodiments, if the user overrides this suggestion, the system may ask for more information as to the reasoning of the medical professional to document why they deviated from the clinical guidelines. Further, diagnosis recommendation 1324 also recommends edits to the tests ordered for a patient, adding in a liver function panel (LFT) test based on clinical guidelines. This can save time by getting all the relevant medical testing done at the right times.

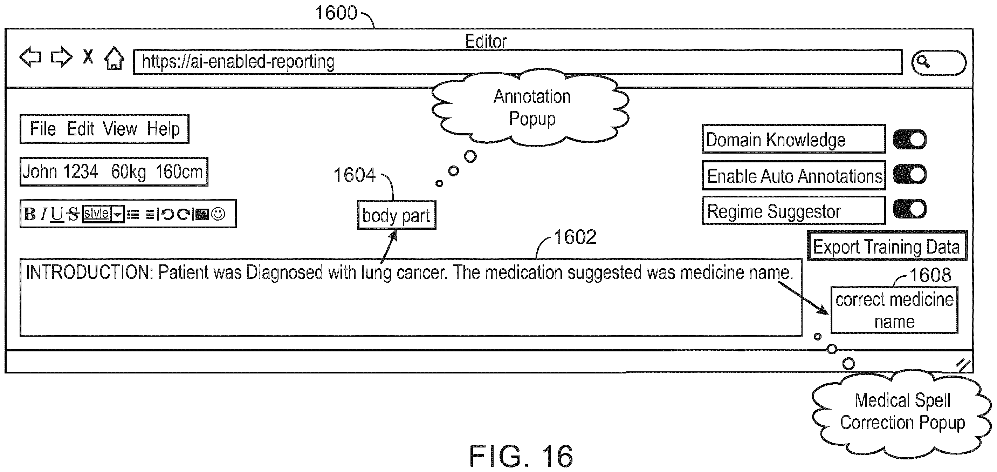

FIG. 16 shows a user interface with suggested edits to a digital document, according to an embodiment. FIG. 16 includes intelligent medical editor 1600 which includes a user interface to interact with a user on a computing device. This may be a personal computer, a smartphone, a tablet, a medical imaging device screen, or other input/output device. FIG. 16 shows medical record 1602 that includes information about a patient. Clinical information extraction module 304, for example, determines that "lung" is a body part 104 and medicine name is a medicine that can be compared by contradiction and incompleteness detection module 312 to verify it is a correct medicine name 1608. This is done through a medical spell correction pop up recommendation and annotation popup, which is different than the above line recommendations shown in FIG. 13. Thus, FIG. 16 shows an intelligent medical editor that is focused on specific domain knowledge the medical field and enables auto annotations and suggestions.

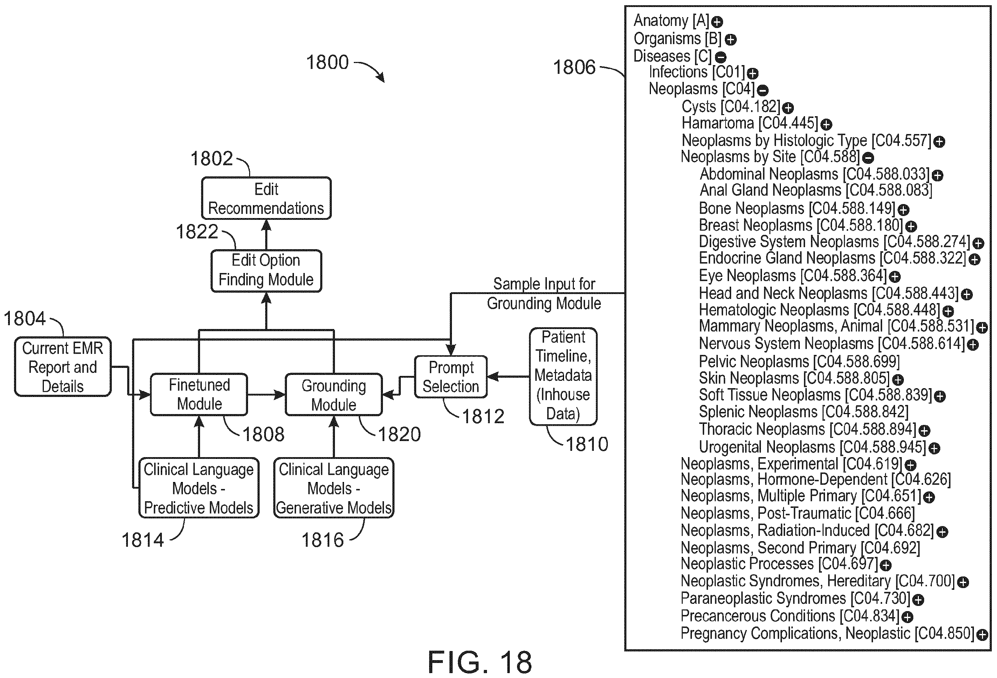

FIG. 18 shows a block diagram related to edit recommendations leveraging predictive and generative models, according to an embodiment. FIG. 18 shows components that can be in an edit recommendation module 1800, edit recommendations 1802, current EMR report and details 1804, medical inputs 1806, finetuned module 1808, patient data 1810, prompt selection 1812, predictive models 1814, generative models 1816, grounding module 1820, and edit option finding module 1822. Input into edit recommendation module 1800 includes medical inputs 1806 that provide a baseline for the grounding module, medical ontologies, medical dictionaries and other medical inputs. In the example of FIG. 18, medical input 1806 includes MeSH tree structure data for medical conditions. The Medical Subject Headings (MeSH) thesaurus is a controlled and hierarchically-organized vocabulary produced by the National Library of Medicine. It is used for indexing, cataloging, and searching of biomedical and health-related information. Input into edit recommendation module 1800 also includes patent data from EMR reports and details 1804 as well as patient data 1810 including timeline metadata, such as from the internal hosted data within a hospital system.

Prompt selection 1812 selects the most relevant information from the multiple options of data suitable for the current report. Finetuned module 1808 receives clinical language predictive models (performing tasks like classification and extraction) and fine tunes them based on feedback and usage information from within the system, and provides finetuned models to grounding module 1820. Finetuned module 1808 picks task specific models based on the current activity of the intelligent medical editor and its users. This may mean finetuned module 1808 picks models specific to the hospital, a disease, or a patient, in various examples. The finetuning process helps the model access external knowledge sources and improve its performance.

Grounding module 1820 takes in fine tuned models from finetuned module 1808, selected information and data from prompt selection 1812, clinical language generative models 1816, metadata, ontologies, clinicians, and other system inputs. Grounding module 1820 determines factual information in the content it generates. This reduces hallucination and inaccuracy issues, and helps minimize randomness that may come from GAI usages. Clinical language generative models 1816 are generative artificial intelligence (GAI), generative language models (GLMs), foundational models, and large language models (LLMs) that perform tasks such as summary generation, text generation, conversational agents, among other tasks. Artificial intelligence (AI) and generative language models (GLMs) present significant opportunities for enhancing medical education, including the provision of realistic simulations, digital patients, personalized feedback, evaluation methods, and the elimination of language barriers. Clinical language generative models 1816 have been specifically trained in the clinical domain.

Edit option finding module 1822 receives fine tuned models and the relevant output from grounding module 1820 to predict options for edits to a medical record, and outputs edit recommendations. Edit recommendations 1802 include providing recommendations, suggesting corrections, and providing alternate text in real time while a report is being types. FIGs. 13-17 show examples of edit recommendations provided to users of an intelligent medical editor.

FIG. 19 shows a flow chart and block diagram related to patient profiling, according to an embodiment. Understanding information regarding a patient is important to an intelligent medical editor. FIG. 19 includes patient profiling system 1900, patient profiler 1902, similar patient finding 1904, editor 1906, future use evaluation 1908, finetuned model selection module 1910, patient data 1912, and past patient data 1914. Patient profiling system 1900 helps gather and understand information about patients.

Patient profiler 1902 builds a context aware patient profile based on patient data throughout the medical systems. This includes a patient EMR data and past TX (treatment/therapy) and DX (diagnosis) related to past patient data 1914. Patient data input into patient profiler also includes patient data 1912 from throughout the medical system including family history, past medical history, examination information, genomic information, drugs, social history, and physical activities. A patient profile can include information such as notable health changes that occur while a patient is at home, doctor or hospital visits, surgical procedures and any related complications, medications, immunizations, like the flu vaccine or tetanus shot, and other related health information. In addition, a user's preferences when working with the medical system technologies, such as a medical system app on their smartphones, an intelligent medical editor, or other computing devices they interact with. Further, patient data can include informal information that can be input via voice, video, personal wearable sensors, and other newer types of input (comments, perspectives, location data and spoken words - from smartphone, security cameras, and the like) as well as observations from friends and other third parties (such as did the patient take the medicine). Patient profiler 1902 is routinely updated to maintain context and history of the patient.

Intelligent medical editor systems and methods can use patient profiles for edit recommendations, contradiction detection, and other aspects discussed throughout. Editor 1906 may provide suggestions and edit recommendations to a user of editor 1906 using patient profile information as well as information related to similar patients (such as through similar patient finding 1904), similar reports, and similar diseases. Patient profiles and similar patient information can be absorbed (intake) into modules throughout the system as part of future use evaluation 1908, such as finetuned model selection module 1910.

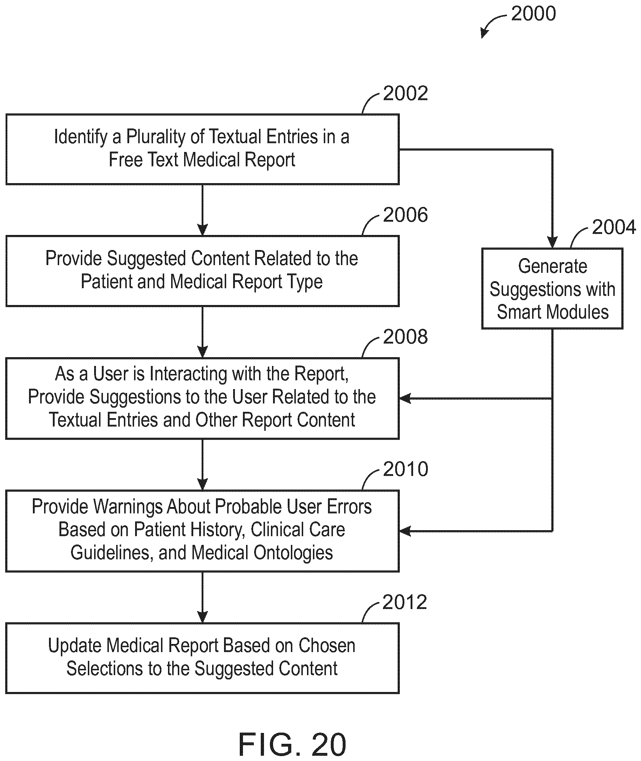

FIG. 20 shows a flow chart for updating a medical report based on selected suggested edits, according to an embodiment. Medical process 2000 may include one or more of six steps that may be performed by an intelligent medical editor. In step 2002, the system may identify a plurality of textual entries in a free text medical report. In step 2004, the system may generate suggestions with smart modules, such as detailed herein throughout and with examples at least related to FIG. 3. In step 2006, the system may provide suggested content related to the patient and medical report type. In step 2008, as a user is interacting with the report, the system may provide suggestions (e.g., edit recommendations) to the user related to the textual entries and other report content. Examples of this are shown in FIGs. 13-17 as examples in various embodiments. In step 2010, the system may provide warnings about probable errors (user or system generated errors) based on patient history, clinical care guidelines, medical ontologies, and other inputs and models. In step 2012, the system may update the medical report based on the chosen selections to the suggested content. Further, the system may update its learning modules (those that allow feedback input based on rules engines, artificial intelligence, generative artificial intelligence, machine learning, deep learning, and the like).

Intelligent medical editor systems and methods allow for medical materials that are correct, timely, and industry best. Intelligent medical editor systems and methods can leverage current government guidance and industry trends in improving healthcare. Intelligent medical editor systems and methods can provide single solution to work with multi-modal time spaced data sources, use of patient histories and by-product modules providing processed data that can be leveraged for other downstream solutions, insights from clinician interactions to assist better with time and provide similar patient profiles for inpatients by using the vast patient histories. Overall patients benefit from previous cases and customized care, clinicians get better assistance and save more time to focus on the treatment and the hospitals benefit by getting one solution for EMR rather than using multiple tools for handling different data, reporting standards, etc. All this is done while maintaining the patient privacy.

Intelligent medical editor systems and methods help ensure the correctness, completeness and clarity of EMRs and other medical records, which improves clinical outcomes. Imprecise and incomplete EMRs adversely affect the speed and quality of all the subsequent clinical decisions such as diagnosis/treatment decisions, clinical trial selection, referencing guidelines and case studies. Intelligent medical editor systems and methods help by providing in-place AI assistance which provide editorial feedback and improvement suggestions to clinician while they are creating and updating an EMR.

Intelligent medical editor systems and methods ensure that the AI models are performing adequately to meet clinical standards is crucial before integrating them with a critical clinical system like EMR. High volume, variety and veracity of manual feedback is essential for validating and continuously improving the AI models. The two roadblocks which hinder such a collection of feedback are (1) the high time and cost of high-quality manual feedback, (2) the data privacy requirements which result in administrative and data anonymization overheads. Intelligent medical editor systems and methods help (1) creating a low-intrusion way for clinicians to provide in-place feedback to AI as well as by (2) creating a federated method to improve the AI models which eliminate the need to share patient data outside of the organization/legal boundaries, thereby enabling wide crowdsourcing of training/validation data for AI models.

Intelligent medical editor systems and methods helps users by pre-annotating a report accurately before clinician opens it, and present this pre-annotation in a readily comprehensible and easily modifiable view to the clinician. Both the pre-annotations (including prepopulated information) and the clinician modified/created annotations help structurally enrich the report with codified concepts. This has following clinical benefits, bring much needed standardization to clinical report. It can be difficult for other users to quickly read other doctor's reports due to them not following synoptic reporting standards required for oncology. Real-time autocorrections and suggestions coming out of NLP subsystem helps in this. Intelligent medical editor systems and methods are useful also as they auto-fill of concepts related to medical standards as mentioned throughout (including FHIR, UB-04 and the like). If the codified concepts can be parsed directly from report, with integration with EMR systems, systems and methods herein can autofill such data, which is a much needed feature both for productivity of doctors as well as enormous benefit to downstream applications.

The systems and processes described below can be embodied within hardware, such as a single integrated circuit (IC) chip, multiple ICs, an application specific integrated circuit (ASIC), or the like. Further, the order in which some or all of the process blocks appear in each process should not be deemed limiting. Rather, it should be understood that some of the process blocks can be executed in a variety of orders, not all of which may be explicitly illustrated in this disclosure.

The illustrated aspects of the disclosure may also be practiced in distributed computing environments where certain tasks are performed by remote processing devices that are linked through a communications network. In a distributed computing environment, program modules can be located in both local and remote memory storage devices.

Moreover, it is to be appreciated that various components described in this description can include electrical circuit(s) that can include components and circuitry elements of suitable value in order to implement the embodiments of the subject innovation(s). Furthermore, it can be appreciated that many of the various components can be implemented on one or more integrated circuit (IC) chips. For example, in one embodiment, a set of components can be implemented in a single IC chip. In other embodiments, one or more of respective components are fabricated or implemented on separate IC chips.

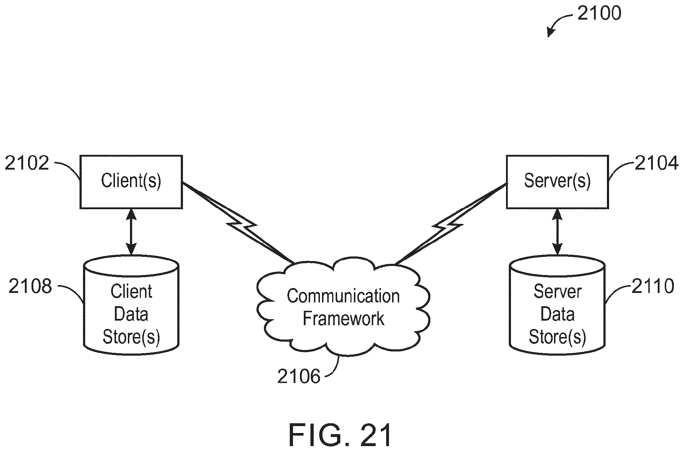

Referring to FIG. 21, there is illustrated a schematic block diagram of a computing environment 2100 in accordance with this disclosure in which the subject systems, methods and computer readable media can be deployed. The computing environment 2100 includes one or more client(s) 2102 (e.g., laptops, smart phones, PDAs, media players, computers, portable electronic devices, tablets, and the like). The client(s) 2102 can be hardware and/or software (e.g., threads, processes, computing devices). The computing environment 2100 also includes one or more server(s) 2104. The server(s) 2104 can also be hardware or hardware in combination with software (e.g., threads, processes, computing devices). The servers 2104 can house threads to perform transformations by employing aspects of this disclosure, for example. In various embodiments, one or more of the subject front end-components can be deployed as hardware and/or software at a client 2102 and one or more of the subject back-end components can be deployed as hardware and/or software at server 2104. One possible communication between a client 2102 and a server 2104 can be in the form of a data packet transmitted between two or more computer processes wherein the data packet may include video data. The data packet can include a metadata, e.g., associated contextual information, for example. The computing environment 2100 includes a communication framework 2106 (e.g., a global communication network such as the Internet, or mobile network(s)) that can be employed to facilitate communications between the client(s) 2102 and the server(s) 2104.

Communications can be facilitated via a wired (including optical fiber) and/or wireless technology. The client(s) 2102 include or are operatively connected to one or more client data store(s) 2108 that can be employed to store information local to the client(s) 2102 (e.g., associated contextual information). Similarly, the server(s) 2104 are operatively include or are operatively connected to one or more server data store(s) 2110 that can be employed to store information local to the servers 2104.

In one embodiment, a client 2102 can transfer an encoded file, in accordance with the disclosed subject matter, to server 2104. Server 2104 can store the file, decode the file, or transmit the file to another client 2102. It is to be appreciated, that a client 2102 can also transfer uncompressed file to a server 2104 and server 2104 can compress the file in accordance with the disclosed subject matter. Likewise, server 2104 can encode video information and transmit the information via communication framework 2106 to one or more clients 2102.

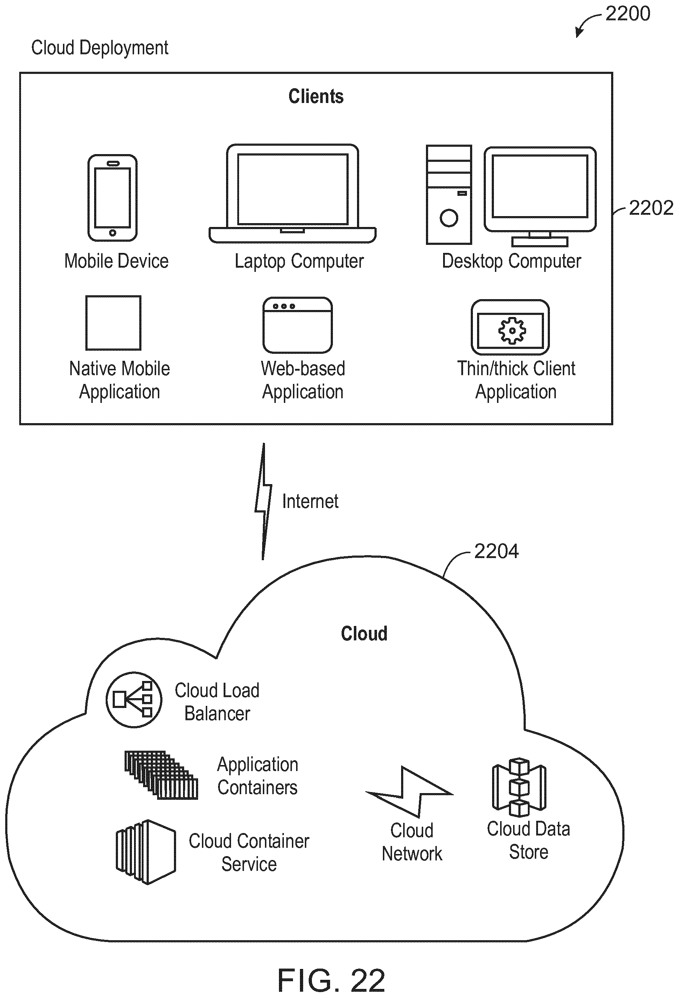

FIG. 22 illustrates a schematic block diagram of another example computing environment 2200 in accordance with this disclosure in which the subject systems, methods and computer readable media can be deployed. The computing environment 2200 includes a cloud deployment architecture consisting of one or more clients 2202 that can be communicatively coupled to a system cloud 2204 via a network (e.g., the Internet). The system cloud 2204 can include a cloud load balances, one or more application container, one or more cloud service containers, a cloud data store, and a cloud network that communicatively couples the one or more cloud components to the cloud data store. In accordance with the cloud deployment architecture, the clients 2202 can include one or more clients devices (e.g., a mobile device, a laptop computer, a desktop computer, etc.) which can include or employ a suitable application (e.g., a native mobile application, a web-based application, a thin/thick client application, etc.) to access and employ one or more features and functionalities of the subject native/reconstructed medical imaging systems deployed in the system cloud 2204. In various implementations, the one or more components of system 100 can be distributed between the clients 2202 and the system cloud 2204.

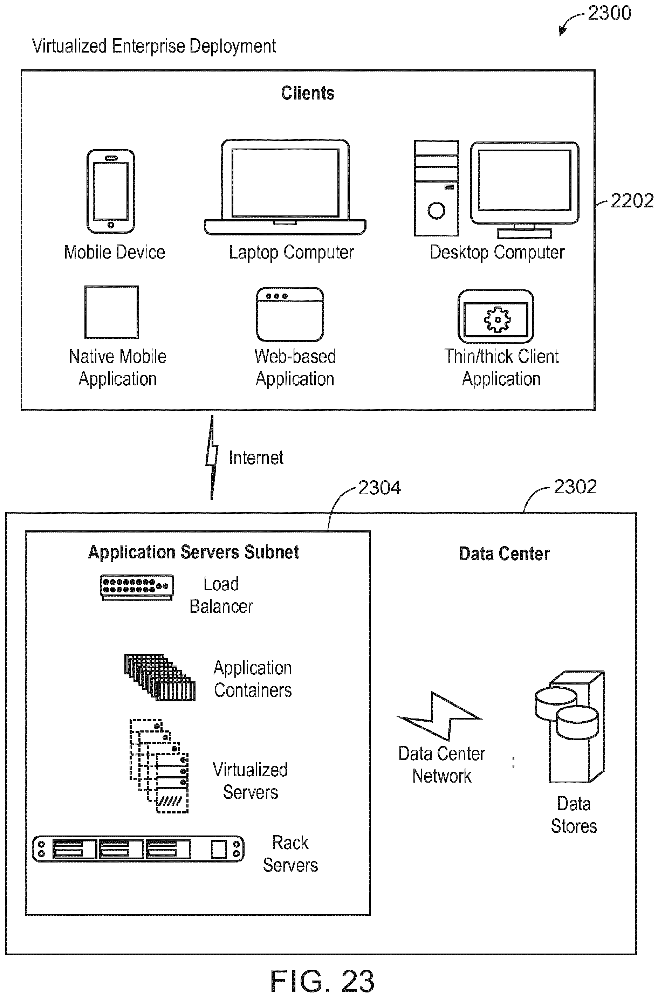

FIG. 23 illustrates a schematic block diagram of another example computing environment 2300 in accordance with this disclosure in which the subject systems, methods and computer readable media can be deployed. The computing environment 2300 includes a virtualized enterprise deployment consisting of one or more clients 2202 that can be communicatively coupled to a remote data center 2302 via a network (e.g., the Internet). The remote data center 2302 can include an application servers subnet 2304 which can provide a load balancer, one or more application containers, one or more virtualized servers and one or more rack servers. The data center 2302 can also include one or more data stores that can be communicatively coupled to the application servers subnet 2304 via a data center network. In accordance with the virtualized enterprise deployment, the clients 2202 can include one or more clients devices (e.g., a mobile device, a laptop computer, a desktop computer, etc.) which can include or employ a suitable application (e.g., a native mobile application, a web-based application, a thin/thick client application, etc.) to access and employ one or more features and functionalities of the subject native/reconstructed medical imaging systems deployed in the data center 2302 and application servers subnet 2304. In various implementations, the one or more components of systems 100 can be distributed between the clients 2202 and the application servers subnet 2304 and the one or more data stores can be provided remotely at the data center 2302.

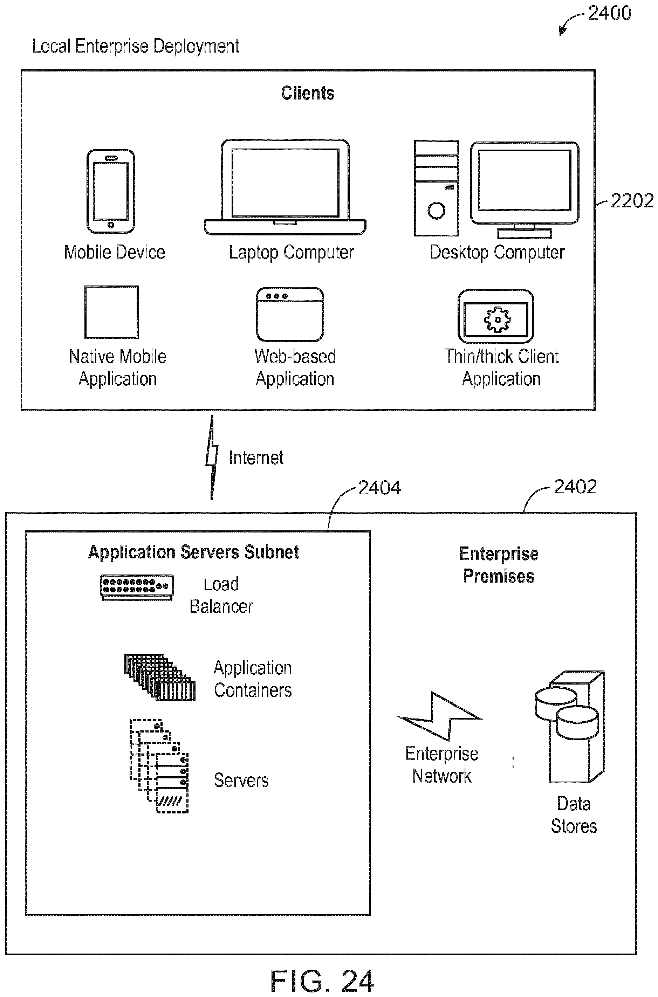

FIG. 24 illustrates a schematic block diagram of another example computing environment 2400 in accordance with this disclosure in which the subject systems, methods and computer readable media can be deployed. The computing environment 2400 includes a local enterprise deployment consisting of one or more clients 2202 that can be communicatively coupled to an application servers subnet 2404 via a network (e.g., the Internet). In accordance with this embodiment, the application servers subnet 2404 can be provided at the enterprise premises 2402 (e.g., as opposed to a remote data center 2302). The application servers subnet 2404 can include a load balancer, one or more application containers and one or more servers. The application servers subnet 2404 can be communicatively coupled to one or more data stores provided at the enterprise premises 2402 via an enterprise network. Similar to the cloud and virtualized enterprise deployments, the clients 2202 can include one or more clients devices (e.g., a mobile device, a laptop computer, a desktop computer, etc.) which can include or employ a suitable application (e.g., a native mobile application, a web-based application, a thin/thick client application, etc.) to access and employ one or more features and functionalities of the subject native/reconstructed medical imaging systems (e.g., system 100and the like) deployed at the enterprise premises 2402 and the application servers subnet 2404. In various implementations, the one or more components of systems herein can be distributed between the clients 2202 and the application servers subnet 2404 and the one or more data stores can be provided at the enterprise premises 2402.

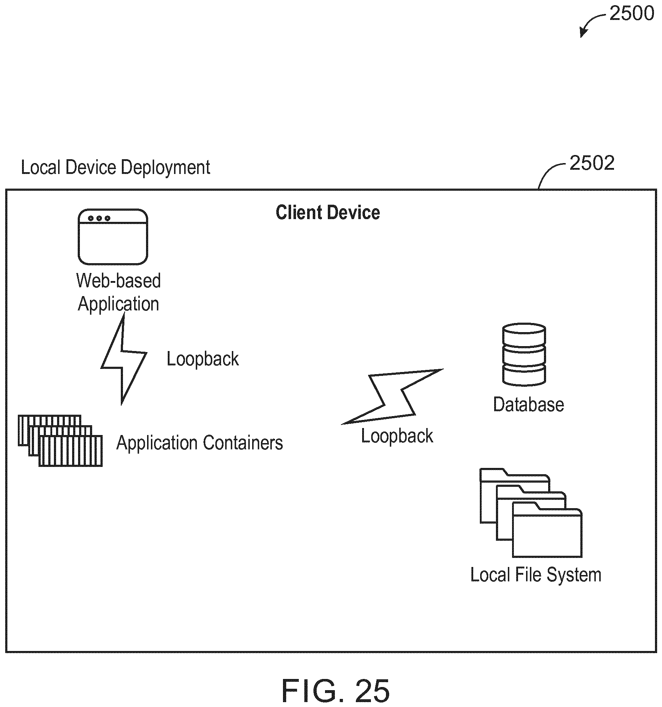

FIG. 25 illustrates a schematic block diagram of another example computing environment in accordance with this disclosure in which the subject systems, methods and computer readable media can be deployed. The computing environment includes a local device deployment in which all of the components of systems herein are provided at a single client device 2502. With this implementation, the client device 2502 can include a web-based application which can be communicatively coupled via a loopback to one or more application containers. The one or more application containers can be communicatively coupled via a loopback to one or more databases and/or one or more local file systems.

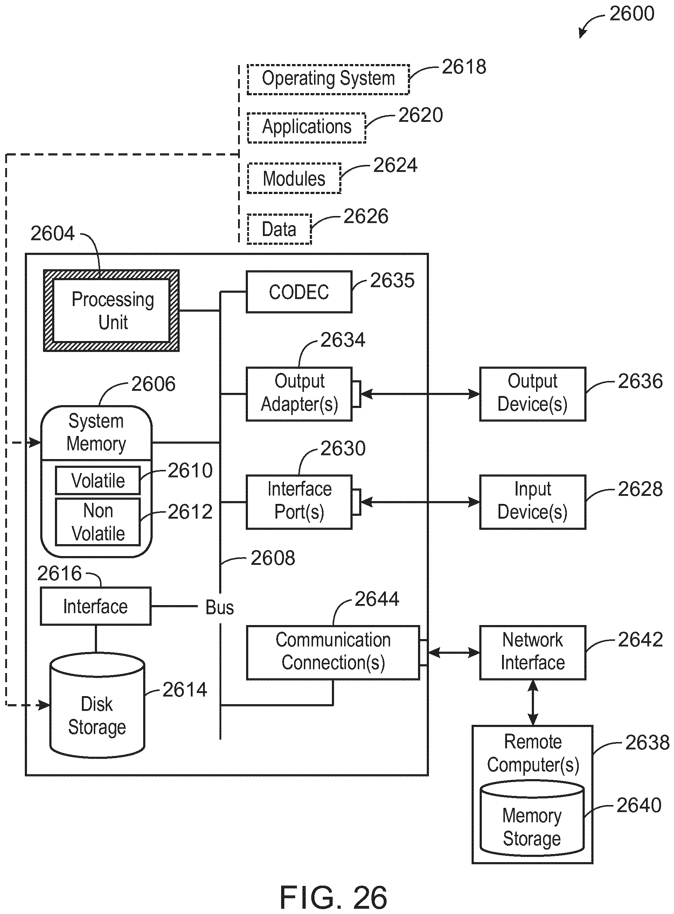

With reference to FIG. 26, a suitable environment 2600 for implementing various aspects of the claimed subject matter includes a computer 2602. The computer 2602 includes a processing unit 2604, a system memory 2606, a codec 2605, and a system bus 2608. The system bus 2608 couples system components including, but not limited to, the system memory 2606 to the processing unit 2604. The processing unit 2604 can be any of various available processors. Dual microprocessors and other multiprocessor architectures also can be employed as the processing unit 2604.

The system bus 2608 can be any of several types of bus structure(s) including the memory bus or memory controller, a peripheral bus or external bus, and/or a local bus using any variety of available bus architectures including, but not limited to, Industrial Standard Architecture (ISA), Micro-Channel Architecture (MSA), Extended ISA (EISA), Intelligent Drive Electronics (IDE), VESA Local Bus (VLB), Peripheral Component Interconnect (PCI), Card Bus, Universal Serial Bus (USB), Advanced Graphics Port (AGP), Personal Computer Memory Card International Association bus (PCMCIA), Firewire (IEEE 22104), and Small Computer Systems Interface (SCSI).

The system memory 2606 includes volatile memory 2610 and non-volatile memory 2612. The basic input/output system (BIOS), containing the basic routines to transfer information between elements within the computer 2602, such as during start-up, is stored in non-volatile memory 2612. In addition, according to present innovations, codec 2605 may include at least one of an encoder or decoder, wherein the at least one of an encoder or decoder may consist of hardware, a combination of hardware and software, or software. Although, codec 2605 is depicted as a separate component, codec 2605 may be contained within non-volatile memory 2612. By way of illustration, and not limitation, non-volatile memory 2612 can include read only memory (ROM), programmable ROM (PROM), electrically programmable ROM (EPROM), electrically erasable programmable ROM (EEPROM), or flash memory. Volatile memory 2210 includes random access memory (RAM), which acts as external cache memory. According to present aspects, the volatile memory may store the write operation retry logic and the like. By way of illustration and not limitation, RAM is available in many forms such as static RAM (SRAM), dynamic RAM (DRAM), synchronous DRAM (SDRAM), double data rate SDRAM (DDR SDRAM), and enhanced SDRAM (ESDRAM).

Computer 2602 may also include removable/non-removable, volatile/non-volatile computer storage medium. FIG. 19 illustrates, for example, disk storage 2614. Disk storage 2614 includes, but is not limited to, devices like a magnetic disk drive, solid state disk (SSD) floppy disk drive, tape drive, Zip drive, flash memory card, or memory stick. In addition, disk storage 2614 can include storage medium separately or in combination with other storage medium including, but not limited to, an optical disk drive such as a compact disk ROM device (CD-ROM), CD recordable drive (CD-R Drive), CD rewritable drive (CD-RW Drive) or a digital versatile disk ROM drive (DVD-ROM). To facilitate connection of the disk storage devices 2614 to the system bus 2608, a removable or non-removable interface is typically used, such as interface 2616.

It is to be appreciated that FIG. 26 describes software that acts as an intermediary between users and the basic computer resources described in the suitable operating environment 2600. Such software includes an operating system 2618. Operating system 2618, which can be stored on disk storage 2614, acts to control and allocate resources of the computer system 2602. Applications 2620 take advantage of the management of resources by operating system 2618 through program modules 2624, and program data 2626, such as the boot/shutdown transaction table and the like, stored either in system memory 2606 or on disk storage 2614. It is to be appreciated that the claimed subject matter can be implemented with various operating systems or combinations of operating systems.

A user enters commands or information into the computer 2602 through input device(s) 2628. Input devices 2628 include, but are not limited to, a pointing device such as a mouse, trackball, stylus, touch pad, keyboard, microphone, joystick, game pad, satellite dish, scanner, TV tuner card, digital camera, digital video camera, web camera, microphone, and the like. These and other input devices connect to the processing unit 2604 through the system bus 2608 via interface port(s) 2630. Interface port(s) 2630 include, for example, a serial port, a parallel port, a game port, and a universal serial bus (USB). Output device(s) 2636 use some of the same type of ports as input device(s). Thus, for example, a USB port may be used to provide input to computer 2602, and to output information from computer 2602 to an output device 2636. Output adapter 2634 is provided to illustrate that there are some output devices 2636 like monitors, speakers, and printers, among other output devices 2636, which require special adapters. The output adapters 2634 include, by way of illustration and not limitation, video and sound cards that provide a means of connection between the output device 2636 and the system bus 2608. It should be noted that other devices and/or systems of devices provide both input and output capabilities such as remote computer(s) 2638.

Computer 2602 can operate in a networked environment using logical connections to one or more remote computers, such as remote computer(s) 2638. The remote computer(s) 2638 can be a personal computer, a server, a router, a network PC, a workstation, a microprocessor based appliance, a peer device, a smart phone, a tablet, or other network node, and typically includes many of the elements described relative to computer 2602. For purposes of brevity, only a memory storage device 2640 is illustrated with remote computer(s) 2638. Remote computer(s) 2638 is logically connected to computer 2602 through a network interface 2642 and then connected via communication connection(s) 2644. Network interface 2642 encompasses wire and/or wireless communication networks such as local-area networks (LAN) and wide-area networks (WAN) and cellular networks. LAN technologies include Fiber Distributed Data Interface (FDDI), Copper Distributed Data Interface (CDDI), Ethernet, Token Ring and the like. WAN technologies include, but are not limited to, point-to-point links, circuit switching networks like Integrated Services Digital Networks (ISDN) and variations thereon, packet switching networks, and Digital Subscriber Lines (DSL).

Communication connection(s) 2644 refers to the hardware/software employed to connect the network interface 2642 to the bus 2608. While communication connection 2644 is shown for illustrative clarity inside computer 2602, it can also be external to computer 2602. The hardware/software necessary for connection to the network interface 2642 includes, for exemplary purposes only, internal and external technologies such as, modems including regular telephone grade modems, cable modems and DSL modems, ISDN adapters, and wired and wireless Ethernet cards, hubs, and routers.

What has been described above includes examples of the embodiments of the present invention. It is, of course, not possible to describe every conceivable combination of components or methodologies for purposes of describing the claimed subject matter, but it is to be appreciated that many further combinations and permutations of the subject innovation are possible. Accordingly, the claimed subject matter is intended to embrace all such alterations, modifications, and variations that fall within the spirit and scope of the appended claims. Moreover, the above description of illustrated embodiments of the subject disclosure, including what is described in the Abstract, is not intended to be exhaustive or to limit the disclosed embodiments to the precise forms disclosed. While specific embodiments and examples are described in this disclosure for illustrative purposes, various modifications are possible that are considered within the scope of such embodiments and examples, as those skilled in the relevant art can recognize.

In particular and in regard to the various functions performed by the above described components, devices, circuits, systems and the like, the terms used to describe such components are intended to correspond, unless otherwise indicated, to any component which performs the specified function of the described component (e.g., a functional equivalent), even though not structurally equivalent to the disclosed structure, which performs the function in the disclosure illustrated exemplary aspects of the claimed subject matter. In this regard, it will also be recognized that the innovation includes a system as well as a computer-readable storage medium having computer-executable instructions for performing the acts and/or events of the various methods of the claimed subject matter.

The aforementioned systems/circuits/modules have been described with respect to interaction between several components/blocks. It can be appreciated that such systems/circuits and components/blocks can include those components or specified sub-components, some of the specified components or sub-components, and/or additional components, and according to various permutations and combinations of the foregoing. Sub-components can also be implemented as components communicatively coupled to other components rather than included within parent components (hierarchical). Additionally, it should be noted that one or more components may be combined into a single component providing aggregate functionality or divided into several separate sub-components, and any one or more middle layers, such as a management layer, may be provided to communicatively couple to such sub-components in order to provide integrated functionality. Any components described in this disclosure may also interact with one or more other components not specifically described in this disclosure but known by those of skill in the art.

In addition, while a particular feature of the subject innovation may have been disclosed with respect to only one of several implementations, such feature may be combined with one or more other features of the other implementations as may be desired and advantageous for any given or particular application. Furthermore, to the extent that the terms "includes," "including," "has," "contains," variants thereof, and other similar words are used in either the detailed description or the claims, these terms are intended to be inclusive in a manner similar to the term "comprising" as an open transition word without precluding any additional or other elements.

As used in this application, the terms "component," "system," or the like are generally intended to refer to a computer-related entity, either hardware (e.g., a circuit), a combination of hardware and software, software, or an entity related to an operational machine with one or more specific functionalities. For example, a component may be, but is not limited to being, a process running on a processor (e.g., digital signal processor), a processor, an object, an executable, a thread of execution, a program, and/or a computer. By way of illustration, both an application running on a controller and the controller can be a component. One or more components may reside within a process and/or thread of execution and a component may be localized on one computer and/or distributed between two or more computers. Further, a "device" can come in the form of specially designed hardware; generalized hardware made specialized by the execution of software thereon that enables the hardware to perform specific function; software stored on a computer readable storage medium; software transmitted on a computer readable transmission medium; or a combination thereof.

Moreover, the words "example" or "exemplary" are used in this disclosure to mean serving as an example, instance, or illustration. Any aspect or design described in this disclosure as "exemplary" is not necessarily to be construed as preferred or advantageous over other aspects or designs. Rather, use of the words "example" or "exemplary" is intended to present concepts in a concrete fashion. As used in this application, the term "or" is intended to mean an inclusive "or" rather than an exclusive "or". That is, unless specified otherwise, or clear from context, "X employs A or B" is intended to mean any of the natural inclusive permutations. That is, if X employs A; X employs B; or X employs both A and B, then "X employs A or B" is satisfied under any of the foregoing instances. In addition, the articles "a" and "an" as used in this application and the appended claims should generally be construed to mean "one or more" unless specified otherwise or clear from context to be directed to a singular form.

Computing devices typically include a variety of media, which can include computer-readable storage media and/or communications media, in which these two terms are used in this description differently from one another as follows. Computer-readable storage media can be any available storage media that can be accessed by the computer, is typically of a non-transitory nature, and can include both volatile and nonvolatile media, removable and non-removable media. By way of example, and not limitation, computer-readable storage media can be implemented in connection with any method or technology for storage of information such as computer-readable instructions, program modules, structured data, or unstructured data. Computer-readable storage media can include, but are not limited to, RAM, ROM, EEPROM, flash memory or other memory technology, CD ROM, digital versatile disk (DVD) or other optical disk storage, magnetic cassettes, magnetic tape, magnetic disk storage or other magnetic storage devices, or other tangible and/or non-transitory media which can be used to store desired information. Computer-readable storage media can be accessed by one or more local or remote computing devices, e.g., via access requests, queries or other data retrieval protocols, for a variety of operations with respect to the information stored by the medium.

In view of the exemplary systems described above, methodologies that may be implemented in accordance with the described subject matter will be better appreciated with reference to the flowcharts of the various figures. For simplicity of explanation, the methodologies are depicted and described as a series of acts. However, acts in accordance with this disclosure can occur in various orders and/or concurrently, and with other acts not presented and described in this disclosure. Furthermore, not all illustrated acts may be required to implement the methodologies in accordance with certain aspects of this disclosure. In addition, those skilled in the art will understand and appreciate that the methodologies could alternatively be represented as a series of interrelated states via a state diagram or events. Additionally, it should be appreciated that the methodologies disclosed in this disclosure are capable of being stored on an article of manufacture to facilitate transporting and transferring such methodologies to computing devices. The term article of manufacture, as used in this disclosure, is intended to encompass a computer program accessible from any computer-readable device or storage media

It is to be understood that the above description is intended to be illustrative, and not restrictive. For example, the above-described embodiments (and/or aspects thereof) may be used in combination with each other. In addition, many modifications may be made to adapt a particular situation or material to the teachings of the various embodiments of the invention without departing from their scope. While the dimensions and types of materials described herein are intended to define the parameters of the various embodiments of the invention, the embodiments are by no means limiting and are exemplary embodiments. Many other embodiments will be apparent to those of skill in the art upon reviewing the above description. The scope of the various embodiments of the invention should, therefore, be determined with reference to the appended claims, along with the full scope of equivalents to which such claims are entitled.

In the appended claims, the terms "including" and "in which" are used as the plain-English equivalents of the respective terms "comprising" and "wherein." Moreover, in the following claims, the terms "first," "second," and "third," etc. are used merely as labels, and are not intended to impose numerical requirements on their objects. Further, the limitations of the following claims are not written in means-plus-function format and are not intended to be interpreted based on 35 U.S.C. § 112, sixth paragraph, unless and until such claim limitations expressly use the phrase "means for" followed by a statement of function void of further structure.

This written description uses examples to disclose the various embodiments of the invention, including the best mode, and also to enable any person skilled in the art to practice the various embodiments of the invention, including making and using any devices or systems and performing any incorporated methods. The patentable scope of the various embodiments of the invention is defined by the claims, and may include other examples that occur to those skilled in the art. Such other examples are intended to be within the scope of the claims if the examples have structural elements that do not differ from the literal language of the claims, or if the examples include equivalent structural elements with insubstantial differences from the literal languages of the claims. Embodiments of the present disclosure shown in the drawings and described above are example embodiments only and are not intended to limit the scope of the appended claims, including any equivalents as included within the scope of the claims. Various modifications are possible and will be readily apparent to the skilled person in the art. It is intended that any combination of non-mutually exclusive features described herein are within the scope of the present invention. That is, features of the described embodiments can be combined with any appropriate aspect described above and optional features of any one aspect can be combined with any other appropriate aspect. Similarly, features set forth in dependent claims can be combined with non-mutually exclusive features of other dependent claims, particularly where the dependent claims depend on the same independent claim. Single claim dependencies may have been used as practice in some jurisdictions require them, but this should not be taken to mean that the features in the dependent claims are mutually exclusive.

## Claims

1. A medical system, comprising:
one or more processors;
memory; and
one or more programs, wherein the one or more programs are stored in the memory and configured to be executed by the one or more processors, the one or more programs including instructions for:
identifying a plurality of textual entities in a free-text medical report in real time as a user is writing the report;
suggesting edit recommendations related to the textual entries to the user, where the edit recommendations are generated by one or more artificial intelligence modules; and
wherein the edit recommendation related to probable errors or improvements based on data comprising patient history, clinical care guidelines, and medical ontologies.

2. The medical system of claim 1, further comprising:
retraining the artificial intelligence module using the medical report and the user's selections related to the edit recommendations as a training data.

3. The medical system of claim 1, wherein:
the errors or improvements comprise clinical incorrectness where the report contains findings on absent or incorrect anatomy of the patient.

4. The medical system of claim 1, wherein:
the errors or improvements comprise a treatment plan that is incompatible with the clinical care guidelines.

5. The medical system of claim 1, wherein:
the errors or improvements comprise information in the report about prior medical appointments or prior medical exams that is incomplete.

6. The medical system of claim 1, wherein:
the errors or improvements comprise detected differences between the report and a related synoptic reporting standard related to the report type.

7. The medical system of claim 1, wherein:
the errors or improvements comprise suggested links to source data from the patient EMR or patient images stored in a PACS.

8. The medical system of claim 1, wherein:
edit recommendations are presented on a user interface via a text overlay near the textual entry in the report.

9. The medical system of claim 1, wherein:
edit recommendations are presented on a user interface via a chatbot supported by generative artificial intelligence and large language models.

10. A medical method, performable by a device having one or more processors and memory, including the steps of:
identifying a plurality of textual entities in a free-text medical report in real time as a user is writing the report;
suggesting edit recommendations related to the textual entries to the user, where the edit recommendations are generated by one or more artificial intelligence modules; and
wherein the edit recommendation related to probable errors or improvements based on data comprising patient history, clinical care guidelines, and medical ontologies.

11. The medical method of claim 10, further comprising the step of:
retraining the artificial intelligence module using the medical report and the user's selections related to the edit recommendations as a training data.

12. The medical method of claim 10, wherein:
the errors or improvements comprise clinical incorrectness where the report contains findings on absent or incorrect anatomy of the patient.

13. The medical method of claim 10, wherein:
the errors or improvements comprise a treatment plan that is incompatible with the clinical care guidelines.

14. The medical method of claim 10, wherein:
the errors or improvements comprise information in the report about prior medical appointments or prior medical exams that is incomplete.

15. The medical method of claim 10, wherein:
edit recommendations are presented on a user interface via a chatbot supported by generative artificial intelligence and large language models.
